# EUROPEAN PATENT APPLICATION

(11) **EP 4 585 191 A1**
(43) Date of publication of application: **16.07.2025**
(21) Application number: 23863226.9
(22) Date of filing: 06.09.2023
(51) Int. Cl.: A61F 2/64, A61F 2/68

(54) **JOINT DEVICE, KNEE JOINT DEVICE, JOINT DEVICE CONTROL METHOD, JOINT DEVICE CONTROL PROGRAM, AND STORAGE MEDIUM IN WHICH CONTROL PROGRAM IS STORED**

(30) Priority: 07.09.2022 JP 2022142465
(71) Applicant: HONDA MOTOR CO., LTD., Minato-ku Tokyo 107-8556 (JP)
(72) Inventor: MATSUMOTO Satoki, Wako-shi, Saitama 351-0193 (JP); KOTANI Yoshiaki, Wako-shi, Saitama 351-0193 (JP); ONO Hiromi, Wako-shi, Saitama 351-0193 (JP); OTA Hiroshi, Kawasaki-shi, Kanagawa 212-0013 (JP); OKANO Masahiro, Tokyo 140-0004 (JP)
(74) Representative: Weickmann & Weickmann PartmbB
(86) International application number: PCT/JP2023/032584
(87) International publication number: WO 2024/053688

(57) **Abstract**

A control unit (10) of an electric prosthetic leg (1) controls to be in a first connection state where any one of a first connection and disconnection mechanism (210) and a second connection and disconnection mechanism (220) is connected, during a stance phase which is a weighted state, and controls to be in a disconnection state where the first connection and disconnection mechanism (210) and the second connection and disconnection mechanism (220) are disconnected, or to be in a second connection state where the other of the first connection and disconnection mechanism (210) and the second connection and disconnection mechanism (220) is connected, during a swing phase which is a non-weighted state.

## Description

### TECHNICAL FIELD

The present invention relates to a joint device, a knee joint device, a joint device control method, a joint device control program, and a storage medium storing the control program.

### BACKGROUND ART

In the related art, as an example of a joint device, a prosthetic leg device that is attached to a leg of a robot or a person who has cut his/her leg due to an accident or a disease. For example, Patent Literature 1 discloses an electric prosthetic leg equipped with a transmission having two power transmission paths having different transmission ratios in order to smoothly ascend and descend stairs.

### CITATION LIST

### PATENT LITERATURE

Patent Literature 1: WO2021/251500A

### SUMMARY OF INVENTION

### TECHNICAL PROBLEM

In the joint device equipped with the transmission having two power transmission paths, it is necessary to appropriately switch connection and disconnection of a connection and disconnection device provided on each of the power transmission paths, depending on a state of the joint device. By appropriately switching between the connection and the disconnection of the connection and disconnection device depending on the state of the joint device, the power transmission path is appropriately switched, and convenience and marketability of the joint device are improved.

The present invention is to provide a joint device, a knee joint device, a joint device control method, a joint device control program, and a storage medium storing the control program, which are highly convenient.

### SOLUTION TO PROBLEM

The present invention provides a joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission unit configured to transmit power of the power source,
the power transmission unit has:
   a first power transmission path for transmitting the power at a first transmission ratio; and
   a second power transmission path for transmitting the power at a second transmission ratio different from the first transmission ratio,
the enlarging and reducing device further includes:
   a first connection and disconnection mechanism configured to switch between connection and disconnection of power in the first power transmission path;
   a second connection and disconnection mechanism configured to switch between connection and disconnection of power in the second power transmission path; and
   a control unit configured to control the power source, the first connection and disconnection mechanism, and the second connection and disconnection mechanism,
the joint device is provided to transition between a weighted state where a weight applied from an outside is received and a non-weighted state where no weight is received, and
the control unit,
   (A) when the joint device is in the weighted state,
      controls to be in a first connection state where any one of the first connection and disconnection mechanism and the second connection and disconnection mechanism is connected, and
   (B) when the joint device is in the non-weighted state,
      controls to be in a disconnection state where the first connection and disconnection mechanism and the second connection and disconnection mechanism are disconnected, or
      controls to be in a second connection state where an other of the first connection and disconnection mechanism and the second connection and disconnection mechanism is connected.

The present invention provides a knee joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission unit configured to transmit power of the power source,
the power transmission unit has:
   a first power transmission path for transmitting the power at a first transmission ratio; and
   a second power transmission path for transmitting the power at a second transmission ratio different from the first transmission ratio,
the enlarging and reducing device further includes:
   a first connection and disconnection mechanism configured to switch between connection and disconnection of power in the first power transmission path;
   a second connection and disconnection mechanism configured to switch between connection and disconnection of power in the second power transmission path; and
   a control unit configured to control the power source, the first connection and disconnection mechanism, and the second connection and disconnection mechanism,
the knee joint device is provided to transition between a stance state where a load from a wearer of the knee joint device is received and a swing state where no load is received, and
the control unit,
   (A) when the knee joint device is in the stance state,
      controls to be in a first connection state where any one of the first connection and disconnection mechanism and the second connection and disconnection mechanism is connected, and
   (B) when the knee joint device is in the swing state,
      controls to be in a disconnection state where the first connection and disconnection mechanism and the second connection and disconnection mechanism are disconnected, or
      controls to be in a second connection state where an other of the first connection and disconnection mechanism and the second connection and disconnection mechanism is connected.

The present invention provides a joint device control method for controlling a joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission unit configured to transmit power of the power source,
the power transmission unit has:
   a first power transmission path for transmitting the power at a first transmission ratio; and
   a second power transmission path for transmitting the power at a second transmission ratio different from the first transmission ratio,
the enlarging and reducing device further includes:
   a first connection and disconnection mechanism configured to switch between connection and disconnection of power in the first power transmission path; and
   a second connection and disconnection mechanism configured to switch between connection and disconnection of power in the second power transmission path,
the joint device is provided to transition between a weighted state where a weight applied from an outside is received and a non-weighted state where no weight is received, and
the control method includes the steps of:
   (A) when the joint device is in the weighted state,
      controlling to be in a first connection state where any one of the first connection and disconnection mechanism and the second connection and disconnection mechanism is connected; and
   (B) when the joint device is in the non-weighted state,
      controlling to be in a disconnection state where the first connection and disconnection mechanism and the second connection and disconnection mechanism are disconnected, or
      controlling to be in a second connection state where an other of the first connection and disconnection mechanism and the second connection and disconnection mechanism is connected.

The present invention provides a joint device control program for controlling a joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, in which
the enlarging and reducing device includes a power source, and a power transmission unit configured to transmit power of the power source,
the power transmission unit has:
   a first power transmission path for transmitting the power at a first transmission ratio; and
   a second power transmission path for transmitting the power at a second transmission ratio different from the first transmission ratio,
the enlarging and reducing device further includes:
   a first connection and disconnection mechanism configured to switch between connection and disconnection of power in the first power transmission path; and
   a second connection and disconnection mechanism configured to switch between connection and disconnection of power in the second power transmission path, and
the joint device is provided to transition between a weighted state where a weight applied from an outside is received and a non-weighted state where no weight is received, and
the control program causes a computer to perform the steps of:
   (A) when the joint device is in the weighted state,
      controlling to be in a first connection state where any one of the first connection and disconnection mechanism and the second connection and disconnection mechanism is connected; and
   (B) when the joint device is in the non-weighted state,
      controlling to be in a disconnection state where the first connection and disconnection mechanism and the second connection and disconnection mechanism are disconnected, or
      controlling to be in a second connection state where an other of the first connection and disconnection mechanism and the second connection and disconnection mechanism is connected.

Further, the present invention provides a computer-readable storage medium storing the above-described control program.

### ADVANTAGEOUS EFFECTS OF INVENTION

According to the present invention, the power transmission path is switched by appropriately switching the connection and disconnection of the connection and disconnection device depending on the state of the joint device, and convenience of the joint device is improved.

### BRIEF DESCRIPTION OF DRAWINGS

[FIG. 1] FIG. 1 is a perspective view of an electric prosthetic leg 1 according to a first embodiment as viewed obliquely from the front.
[FIG. 2] FIG. 2 is an exploded perspective view of the electric prosthetic leg 1.
[FIG. 3] FIG. 3 is a cross-sectional view of the electric prosthetic leg 1.
[FIG. 4] FIG. 4 is a cross-sectional view of an expansion-contraction device 140.
[FIG. 5] FIG. 5 is a cross-sectional view of a main part showing a bent state of the electric prosthetic leg 1.
[FIG. 6] FIG. 6 is a cross-sectional view of a main part showing a maximum bent state of the electric prosthetic leg 1.
[FIG. 7] FIG. 7 is a cross-sectional view of a two-way clutch.
[FIG. 8] FIG. 8 is a perspective view of a retainer 282.
[FIG. 9] FIG. 9 is a view showing operations of an operation mechanism 240, in which (A) of FIG. 9 shows a state where a connection and disconnection unit 212 and a connection and disconnection unit 222 are in an off state, and (B) of FIG. 9 shows a state where the connection and disconnection unit 212 is in the off state and the connection and disconnection unit 222 is in an on state, and (C) of FIG. 9 shows a state where the connection and disconnection unit 212 is in the on state and the connection and disconnection unit 222 is in the off state.
[FIG. 10] (A) of FIG. 10 is a cross-sectional view showing a state where the connection and disconnection unit 222 is in the off state, and (B) of FIG. 10 is a view showing a position of an operation rod 241 at that time.
[FIG. 11] (A) of FIG. 11 is a cross-sectional view showing a state where the connection and disconnection unit 222 is operated from the off state to the on state, and (B) of FIG. 11 is a view showing a position of the operation rod 241 at that time.
[FIG. 12] (A) of FIG. 12 is a cross-sectional view showing a normal-rotation on state of the connection and disconnection unit 222, and (B) of FIG. 12 is a view showing a position of the operation rod 241 at that time.
[FIG. 13] (A) of FIG. 13 is a cross-sectional view showing a reverse-rotation on state of the connection and disconnection unit 222, and (B) of FIG. 13 is a view showing a position of the operation rod 241 at that time.
[FIG. 14] (A) of FIG. 14 is a cross-sectional view showing a state where the connection and disconnection unit 222 is operated from the on state to the off state, and (B) of FIG. 14 is a view showing a position of the operation rod 241 at that time.
[FIG. 15] FIG. 15 is a cross-sectional view of the electric prosthetic leg 1 of a second embodiment.
[FIG. 16] FIG. 16 is a functional block diagram of the electric prosthetic leg 1.
[FIG. 17] FIG. 17 is a diagram showing actions (stair ascending actions) of a human and the electric prosthetic leg when ascending stairs.
[FIG. 18] FIG. 18 is a diagram showing actions (flat ground walking actions) of the human and the electric prosthetic leg when walking on a flat ground.
[FIG. 19] (A) of FIG. 19 is a diagram showing a knee angle, (B) of FIG. 19 is a diagram showing a thigh angle, and (C) of FIG. 19 is a diagram showing a lower leg angle.
[FIG. 20] FIG. 20 is a table summarizing phases and corresponding control methods in a stair ascending mode and a flat ground and stair descending mode.
[FIG. 21] FIG. 21 is a graph showing a relationship of the knee angle, the thigh angle, the lower leg angle, and a load in the stair ascending mode.
[FIG. 22] FIG. 22 is a graph showing a relationship of the knee angle, the thigh angle, the lower leg angle, a lower leg angular velocity, and the load in the flat ground and stair descending mode.
[FIG. 23] FIG. 23 is a graph (comparative example) showing the load, the knee angle, and states of a first connection and disconnection mechanism 210 and a second connection and disconnection mechanism 220 when a terminal impact occurs.
[FIG. 24] FIG. 24 is a graph showing the load, the knee angle, and the states of the first connection and disconnection mechanism 210 and the second connection and disconnection mechanism 220 when terminal impact prevent control is performed.
[FIG. 25] FIG. 25 is a graph (comparative example) showing the load, the lower leg angular velocity, and the states of the first connection and disconnection mechanism 210 and the second connection and disconnection mechanism 220 when trip occurs.
[FIG. 26] FIG. 26 is a graph showing the load, the lower leg angular velocity, and the states of the first connection and disconnection mechanism 210 and the second connection and disconnection mechanism 220 when trip prevention control is performed.

### DESCRIPTION OF EMBODIMENTS

Hereinafter, an electric prosthetic leg as an embodiment of a joint device of the present invention will be described below with reference to the drawings. In the following description, a front-rear direction, a left-right direction, and an upper-lower direction are defined with reference to a user of the electric prosthetic leg. In the drawings, a front side of the electric prosthetic leg is denoted by Fr, a rear side is denoted by Rr, a left side is denoted by L, a right side is denoted by R, an upper side is denoted by U, and a lower side is denoted by D.

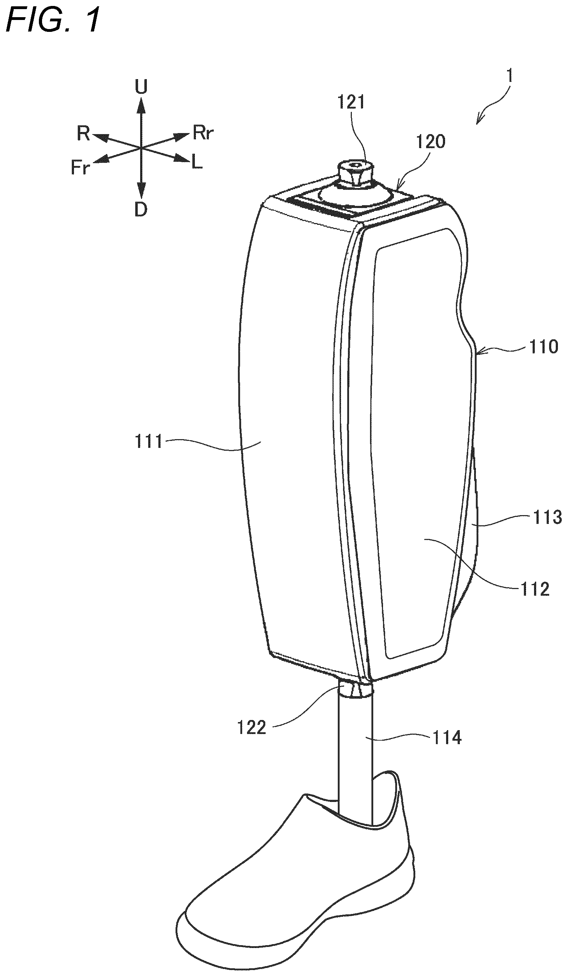

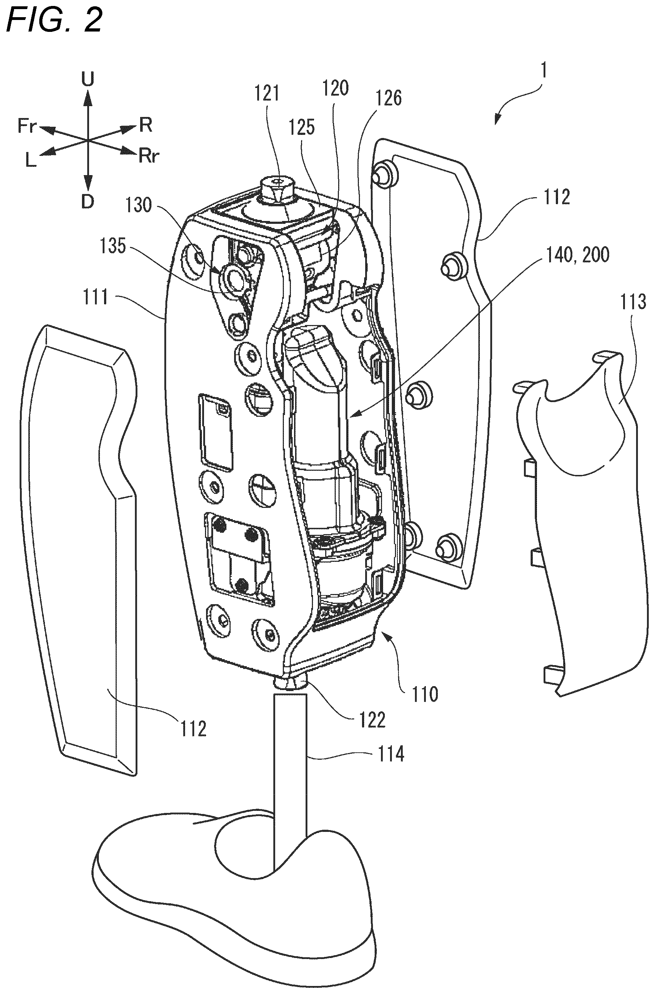

As shown in FIGs. 1 to 4, an electric prosthetic leg 1 according to the present embodiment is a prosthetic leg that is attached to a leg of a person who does not have a knee. The electric prosthetic leg 1 includes: a below-knee member 110 positioned on a lower side of the knee, an above-knee member 120 positioned on an upper side of the knee and attached to a thigh, a knee joint mechanism 130 that couples the below-knee member 110 and the above-knee member 120 such that a formed angle formed therebetween is variable, an enlarging and reducing device 200 capable of enlarging and reducing the formed angle formed between the below-knee member 110 and the above-knee member 120, a mechanical stop mechanism 150 that mechanically limits a range of change in the formed angle formed between the below-knee member 110 and the above-knee member 120, a buffer mechanism 160 that buffers an impact caused by the mechanical stop mechanism 150, a sensor device 270, and a battery B that supplies power to the enlarging and reducing device 200 and the like.

The above-knee member 120 includes an adapter 121 coupled to a socket which is not shown, and an above-knee base portion 126 including an upper wall 125 to which the adapter 121 is attached. The socket is a joint member provided on the thigh, and the above-knee member 120 is integrated with the thigh by coupling the adapter 121 to the socket.

The below-knee member 110 includes a box-shaped main frame 111 with open upper portion and rear portion, side covers 112 that cover both left and right side surfaces of the main frame 111, a detachable rear cover 113 that covers a rear opening of the main frame 111 in an openable and closable manner, and an adapter 122 attached to a lower surface of the main frame 111.

The above-knee member 120 is provided on the upper portion of the main frame 111 of the below-knee member 110 via a coupling axis 135 that constitutes the knee joint mechanism 130, and a leg 114 extending downward is coupled to the adapter 122 of the main frame 111.

An enlarging and reducing device 200 capable of enlarging and reducing the formed angle formed between the above-knee member 110 and the below-knee member 120 is provided in a space formed by the below-knee member 120 and the above-knee member 110. The enlarging and reducing device 200 is an expansion-contraction device 140 capable of enlarging and reducing the formed angle formed between the below-knee member 110 and the above-knee member 120 by expansion and contraction. The expansion-contraction device 140 extends in the upper-lower direction, which will be described later in detail, and is mechanically connected to the above-knee member 120 on one end side in the extending direction and mechanically connected to the below-knee member 110 on the other end side in the extending direction. The term "mechanically connected" is a concept that includes a configuration of direct connection and a configuration of connection via another member.

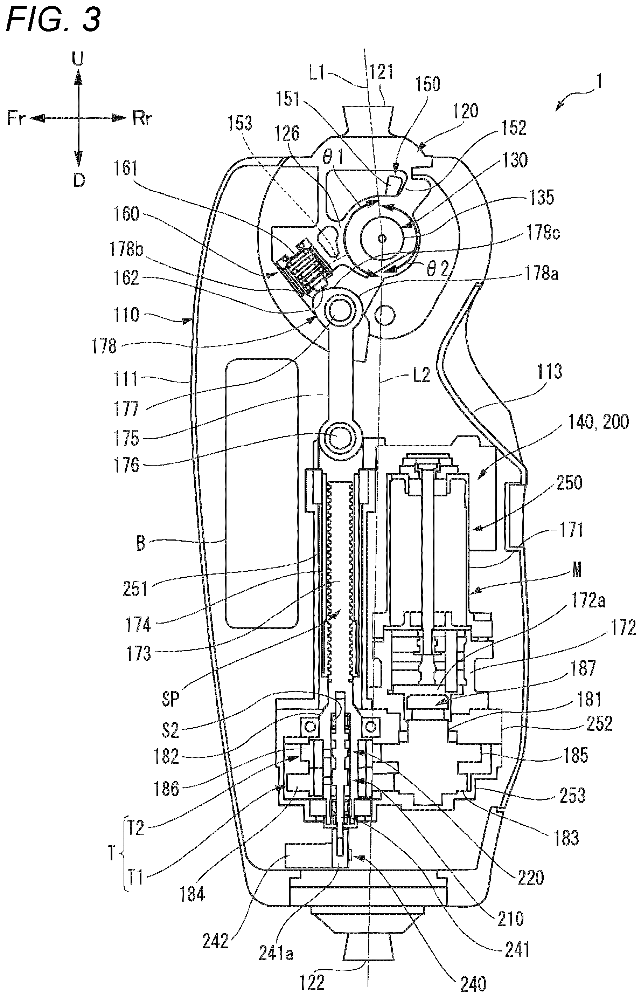

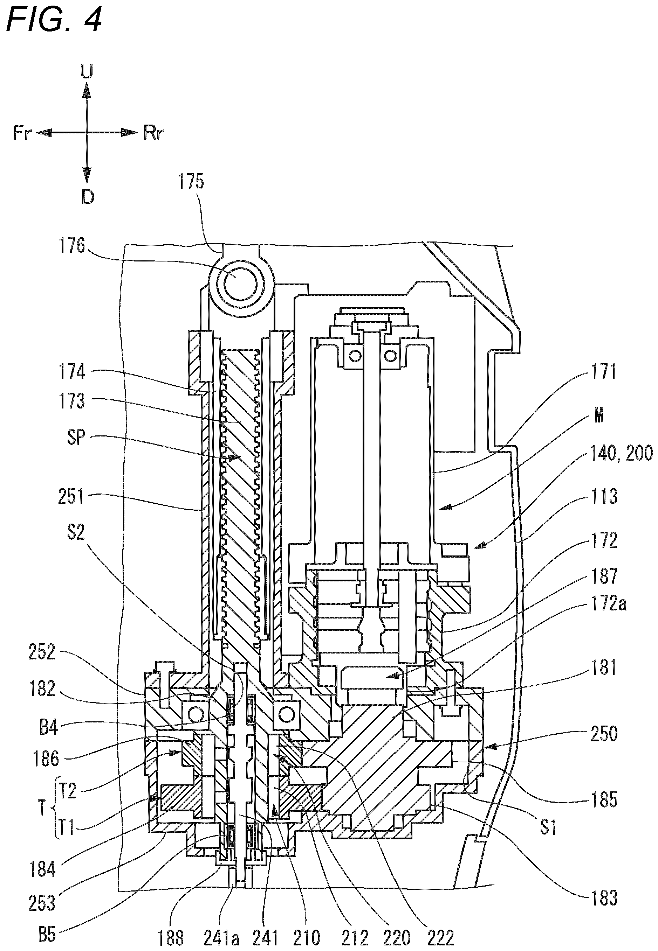

As shown in FIGs. 3 and 4, the expansion-contraction device 140 includes: a motor M that outputs rotational power, a transmission T that transmits the power of the motor M, a spindle unit SP that is connected to the transmission T in a manner of being capable of transmitting power and converts the rotational power output from the transmission T into translational motion (expansion-contraction motion), a first connection and disconnection mechanism 210 and a second connection and disconnection mechanism 220 which are provided in the transmission T, an operation mechanism 240 for switching between the first connection and disconnection mechanism 210 and the second connection and disconnection mechanism 220, and a control unit 10 that controls the motor M, and controls the first connection and disconnection mechanism 210 and the second connection and disconnection mechanism 220 by operating the operation mechanism 240.

The motor M is, for example, a permanent-magnet-type electric motor, and is disposed on the rear side and upper side of the transmission T, and the spindle unit SP is disposed on the front side and upper side of the transmission T. The spindle unit SP is disposed on a side opposite to the motor M with respect to the transmission T on a transmission path of the power. The motor M is a motor with a built-in gear mechanism, including a motor body 171 and a gear mechanism unit 172 that decelerates output rotation of the motor body 171. The spindle unit SP includes a spindle 173 formed with a male screw and a sleeve 174 formed with a female screw, and rotation of the spindle 173 causes the sleeve 174 to perform translational motion along an axis of the spindle 173.

Specifically, the spindle 173 rotates after receiving the rotational power of the motor M transmitted by the transmission T. On the other hand, the sleeve 174 is non-rotatably and movably in the upper-lower direction supported by a unit case 250. Therefore, when the spindle 173 rotates to one side after receiving the rotational power of the motor M transmitted by the transmission T, the sleeve 174 is translated away from the transmission T, and when the spindle 173 rotates to the other side, the sleeve 174 is translated in a direction of approaching the transmission T. The translation operation of the sleeve 174 away from the transmission T may be referred to as an expansion operation of the spindle unit SP, and conversely, the translation operation of the sleeve 174 approaching the transmission T may be referred to as a contraction operation of the spindle unit SP.

That is, a distance between the sleeve 174 and the transmission T increases or decreases depending on a rotation direction of the spindle 173. An upper end of the sleeve 174 is coupled to the above-knee member 120 via a link member 175. As the distance between the sleeve 174 and the transmission T increases or decreases depending on the rotation direction of the spindle 173, the below-knee member 110 and the above-knee member 120 rotate around the coupling axis 135. Accordingly, the formed angle formed between the above-knee member 120 and the below-knee member 110 changes.

Here, the formed angle formed between the above-knee member 120 and the below-knee member 110 is an angle defined by a first virtual line L1 connecting a center of the coupling axis 135 of the knee joint mechanism 130 and the adapter 121 of the above-knee member 120 and a second virtual line L2 extending downward in a vertical direction through the center of the coupling axis 135 of the knee joint mechanism 130 and the below-knee member 110. Among the angles formed between the below-knee member 110 and the above-knee member 120 and centered on the coupling axis 135 of the knee joint mechanism 130, an angle on one side in one circumference is defined as a first formed angle θ1, and an angle on the other side in the circumference is defined as a second formed angle θ2. When a smaller minimum formed angle formed in a range of relative movement between the below-knee member 110 and the above-knee member 120 is, out of the first formed angle θ1 and the second formed angle θ2, defined as the second formed angle θ2, an angle (knee-back angle) formed on a back side of the knee of the user of the electric prosthetic leg 1 is the second formed angle θ2. The first formed angle θ1 takes a value of about 175 [deg] to 300 [deg], and the second formed angle θ2 takes a value of about 60 [deg] to 185 [deg].

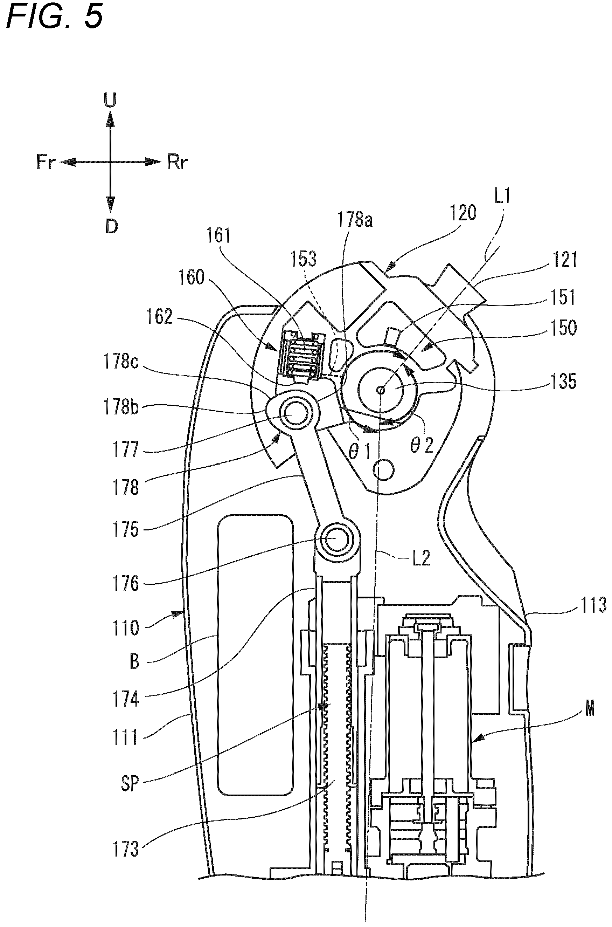

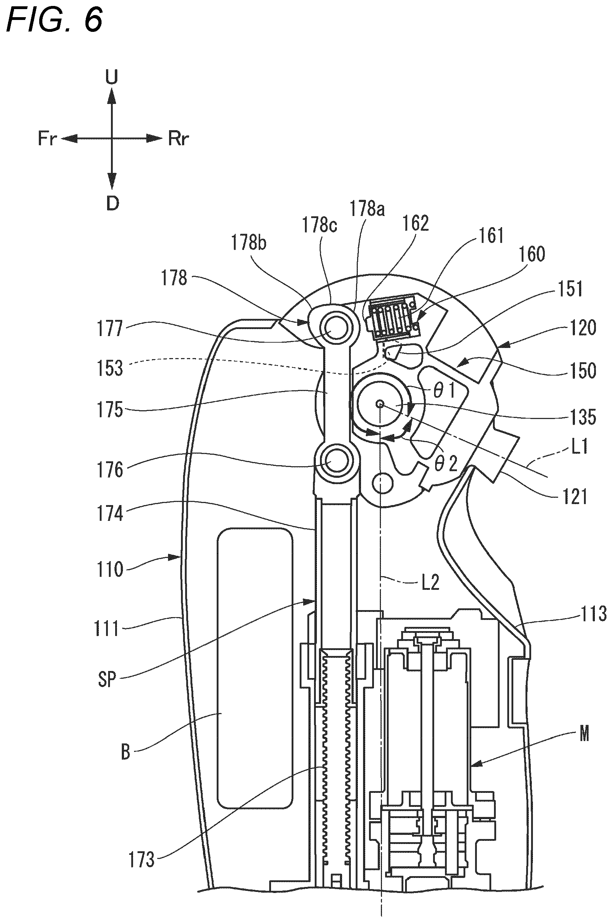

FIG. 3 shows a stretched state of the knee joint mechanism 130, in which the first formed angle θ1 is about 175 [deg], and the second formed angle θ2 is about 185 [deg]. FIG. 5 is a cross-sectional view of a main part showing a bent state of the electric prosthetic leg 1, in which the first formed angle θ1 is about 240 [deg] and the second formed angle θ2 is about 120 [deg]. FIG. 6 is a cross-sectional view of a main part showing a maximum bent state of the electric prosthetic leg 1, in which the first formed angle θ1 is about 300 [deg] and the second formed angle θ2 is about 60 [deg].

The enlarging and reducing device 200 of the present embodiment causes the expansion-contraction device 140 to expand and contract by converting the rotation motion into the expansion-contraction motion by the spindle unit SP of the expansion-contraction device 140, thereby enlarging and reducing the formed angle formed between the below-knee member 110 and the above-knee member 120. However, instead of a portion that expands and contracts (moves) as the expansion-contraction device 140 (spindle unit SP), a gear meshing mechanism (or the like) may be provided between the below-knee member 110 and the above-knee member 120 to enlarge and reduce the formed angle formed between the below-knee member 110 and the above-knee member 120.

Returning to FIGs. 3 and 4, the transmission T includes a first transmission mechanism T1 that includes a first transmission unit transmitting the power of the motor M to the spindle unit SP at a first transmission ratio, and a second transmission mechanism T2 that includes a second transmission unit transmitting the power of the motor M to the spindle unit SP at a second transmission ratio, which is different from the first transmission ratio. The first transmission mechanism T1 and the second transmission mechanism T2 are switched between a power disconnection state and a power connection state by the connection and disconnection mechanisms 210 and 220.

According to such a transmission T, by providing two power transmission paths with different transmission ratios, it is possible to switch an operating speed and generated power for stretching and bending in the knee joint mechanism 130. As long as the first transmission ratio is different from the second transmission ratio, one of the first transmission mechanism T1 and the second transmission mechanism T2 may be a speed reduction mechanism and the other may be a speed increasing mechanism, or one may be a constant speed mechanism and the other may be a speed reduction mechanism or a speed increasing mechanism, or both may be speed reduction mechanisms, or both may be speed increasing mechanisms.

The first transmission ratio is a ratio of a post-transmission rotation speed, which is a rotation speed on a motor M opposite-side (spindle unit SP side) of the first transmission unit in the first transmission mechanism T1, with respect to a pre-transmission rotation speed, which is a rotation speed on a motor M side of the first transmission unit in the first transmission mechanism T1. The second transmission ratio is a ratio of a post-transmission rotation speed, which is a rotation speed on a motor M opposite-side (spindle unit SP side) of a second transmission unit in the second transmission mechanism T2, with respect to a pre-transmission rotation speed, which is a rotation speed on a motor M side of the second transmission unit in the second transmission mechanism T2.

For example, when the first transmission ratio of the first transmission mechanism T1 is smaller than 1, the rotation speed on the motor M opposite-side (spindle unit SP side) becomes lower than the rotation speed on the motor M side, and a torque increases. When the second transmission ratio of the second transmission mechanism T2 is larger than 1, the rotation speed on the motor M opposite-side (spindle unit SP side) becomes higher than the rotation speed on the motor M side, and a torque decreases. In the present embodiment, the first transmission ratio is set to be smaller than 1 and the second transmission ratio is set to be larger than 1, and the first transmission mechanism T1 is disposed below the second transmission mechanism T2.

The first transmission mechanism T1 and the second transmission mechanism T2 include a first shaft 181 rotatably disposed on a downward extension line of an output axis 172a of the gear mechanism unit 172, and a second shaft 182 rotatably disposed on a downward extension line of the spindle 173 of the spindle unit SP. The first shaft 181 is coupled to the output axis 172a of the gear mechanism unit 172 of the motor M in a manner of being integrally rotatable, via a coupling 187 that allows an axis center error. The second shaft 182 is connected to the spindle 173 of the spindle unit SP in a manner of being integrally rotatable. Although the second shaft 182 of the present embodiment is integrated with the spindle 173 of the spindle unit SP, the second shaft 182 may be coupled to the spindle 173 of the spindle unit SP by spline fitting or using a coupling.

The first transmission mechanism T1 includes the first transmission unit including a first drive gear 183 and a first driven gear 184 that mesh with each other. The first drive gear 183 is supported by the first shaft 181 in a manner of being integrally rotatable, and the first driven gear 184 is supported by the second shaft 182 in a manner of being relatively rotatable. The first driven gear 184 and the second shaft 182 have the same rotation axis. The first transmission mechanism T1 of the present embodiment is a deceleration transmission mechanism in which the first drive gear 183 has a diameter smaller than that of the first driven gear 184, and can cause the spindle unit SP to expand and contract at low speed and high torque.

The second transmission mechanism T2 includes the second transmission unit including a second drive gear 185 and a second driven gear 186 that mesh with each other. The second drive gear 185 is supported by the first shaft 181 in a manner of being integrally rotatable, and the second driven gear 186 is supported by the second shaft 182 in a manner of being relatively rotatable. The second driven gear 186 and the second shaft 182 have the same rotation axis. The second transmission mechanism T2 of the present embodiment is an acceleration transmission mechanism in which the second drive gear 185 has a diameter larger than that of the second driven gear 186, and can cause the spindle unit SP to expand and contract at high speed and low torque. In the present embodiment, the second transmission mechanism T2 is disposed above the first transmission mechanism T1, but the second transmission mechanism T2 may be disposed below the first transmission mechanism T1. Although the first shaft 181 and the second shaft 182 of the present embodiment are integrally formed from the beginning, the first shaft 181 and the second shaft 182 may be integrally coupled (combined) after upper and lower gear support portions are formed as separate bodies.

The first connection and disconnection mechanism 210 includes a connection and disconnection unit 212 provided between the first driven gear 184 and the second shaft 182. The second connection and disconnection mechanism 220 includes a connection and disconnection unit 222 provided between the second driven gear 186 and the second shaft 182. These connection and disconnection units 212 and 222 have a common configuration, and are configured to be switched between a disconnection state where power transmission is disconnected, and a power transmittable state where rotational power in both one direction and the other direction can be transmitted. Details of the connection and disconnection units 212 and 222 will be described later.

The operation mechanism 240 includes an operation rod 241 configured to intermittently operate the connection and disconnection units 212 and 222, and a servo motor 242 that linearly moves the operation rod 241.

The second shaft 182 is a hollow shaft having an internal space S2 extending in a rotation axis direction (also referred to as the upper-lower direction), and the operation rod 241 is disposed in the internal space S2. The operation rod 241 is provided with a rack 241a at a lower end exposed from the internal space S2. The operation rod 241 is supported by bearings B4 and B5 disposed in the internal space S2 in a manner of not being relatively rotatable with respect to the rack 241a and being capable of integrally advancing and retracting with the rack 241a in the rotation axis direction. A lid member 188 having an insertion hole through which the operation rod 241 is inserted is screwed to a lower end of the second shaft 182. The lid member 188 prevents foreign matters from entering the internal space S2 and facilitates replacement of the operation rod 241. A pinion 243 provided on an output axis 242a of the servo motor 242 meshes with the rack 241a, and a position of the operation rod 241 in the upper-lower direction is switched according to the drive of the servo motor 242. Small-diameter portions 241b1 and 241b2 and large-diameter portions 241c1 to 241c3, which will be described later, are formed on an outer peripheral portion of the operation rod 241, and the small-diameter portions 241b1 and 241b2 and the large-diameter portions 241c1 to 241c3 intermittently operate the connection and disconnection units 212 and 222 according to the position of the operation rod 241. Details of the operation mechanism 240 will be described later.

As shown in FIGs. 3 to 5, the unit case 250 includes an upper case 251, a middle case 252, and a lower case 253. The upper case 251, the middle case 252, and the lower case 253 are formed separately from each other.

The upper case 251 accommodates the spindle unit SP.

A space S1 defined by the middle case 252 and the lower case 253 accommodates the second drive gear 185, the second driven gear 186, the first drive gear 183, the first driven gear 184, the connection and disconnection units 212 and 222, and a part of the operation mechanism 240.

With the three-stage structure of the upper case 251, the middle case 252, and the lower case 253, the unit case 250 not only can house the transmission T and the spindle unit SP, but also can unitize the expansion-contraction device 140 including the motor M.

Further, the unit case 250 is attached to the main frame 111 via a bracket which is not shown.

As shown in FIGs. 3, 5, and 6, the mechanical stop mechanism 150 includes a stopper member 151 provided on the below-knee member 110, and a first contact portion 152 and a second contact portion 153 provided on the above-knee base portion 126 of the above-knee member 120. In a state shown in FIG. 3 (the second formed angle θ2 is about 185 [deg]), the first contact portion 152 comes into contact with the stopper member 151, thereby restricting the knee joint mechanism 130 from bending to an opposite direction. Further, in a state shown in FIG. 6 (the second formed angle θ2 is about 60 [deg]), the second contact portion 153 comes into contact with the stopper member 151, thereby restricting the knee joint mechanism 130 from being further bent from the maximum bent state. In walking with the electric prosthetic leg 1, the maximum bent state shown in FIG. 6 does not occur.

The buffer mechanism 160 is provided on an above-knee member 120 side, and includes a pressing portion 162 capable of pressing an upper end of the link member 175 by a biasing force of a spring 161 (for example, a compression coil spring). A lower end of the link member 175 is rotatably coupled to the sleeve 174 of the spindle unit SP via a first pivoting portion 176, and the upper end of the link member 175 is rotatably coupled to the above-knee member 120 via a second pivoting portion 177. A cam portion 178 is formed at the upper end of the link member 175. The cam portion 178 includes a small-diameter outer peripheral portion 178a having a small diameter and centered on the second pivoting portion 177, a large-diameter outer peripheral portion 178b with a long distance from the second pivoting portion 177, and a coupling outer peripheral portion 178c that couples the small-diameter outer peripheral portion 178a and the large-diameter outer peripheral portion 178b without any step.

As shown in FIGs. 5 and 6, in a state where the knee joint mechanism 130 is bent, the pressing portion 162 faces the small-diameter outer peripheral portion 178a of the cam portion 178, and thus the pressing portion 162 and the cam portion 178 are separated from each other. As shown in FIG. 3, when the knee joint mechanism 130 is stretched in response to a contraction operation of the spindle unit SP and approaches a mechanical stop position on a stretching side, as a facing position between the pressing portion 162 and the cam portion 178 moves from the coupling outer peripheral portion 178c to the large-diameter outer peripheral portion 178b, the cam portion 178 comes into contact with the pressing portion 162 and the large-diameter outer peripheral portion 178b pushes the pressing portion 162 against the biasing force of the spring 161. In other words, the cam portion 178 is pressed in a return direction by the biasing force of the spring 161. Accordingly, the biasing force of the spring 161 acts as resistance, and an impact when the first contact portion 152 comes into contact with the stopper member 151 is buffered.

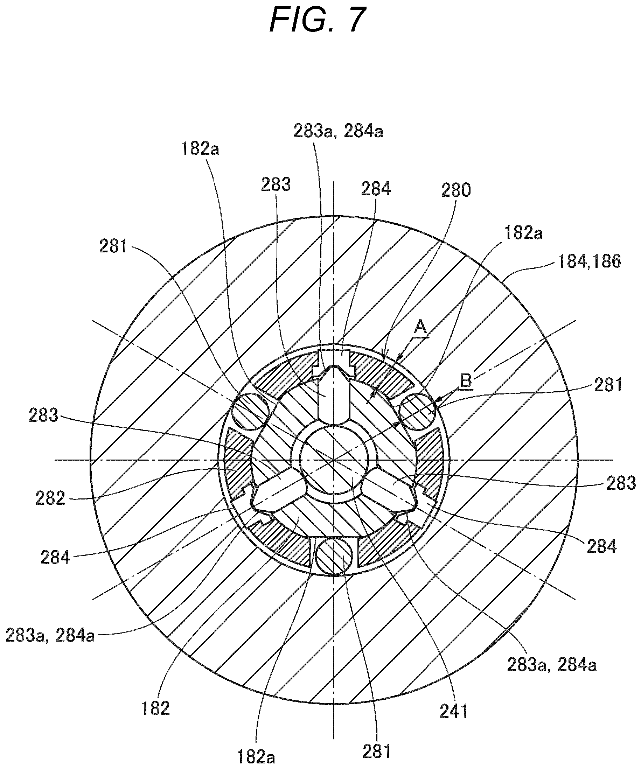

Next, details of the connection and disconnection units 212 and 222 and the operation mechanism 240 will be described with reference to FIG. 7 and subsequent drawings.

The connection and disconnection units 212 and 222 have a common configuration, and are configured to be switched between the disconnection state where the power transmission is disconnected, and the power transmittable state where the rotational power in both one direction and the other direction can be transmitted. Each of the connection and disconnection units 212 and 222 of the present embodiment is configured using a two-way clutch 280 with a forced free function, as shown in FIG. 7. The two-way clutch 280 includes a plurality of (three in the present embodiments) rollers 281 that are arranged between an outer peripheral surface portion of the second shaft 182 and inner peripheral surface portions of the gears 184 and 186, a retainer 282 that holds the plurality of rollers 281 at predetermined intervals, a plurality of (three in the present embodiment) pins 283 that penetrate the second shaft 182 in a radial direction and are operated by the operation mechanism 240 to a forced free position and a forced free release position, and a plurality of (three in the present embodiment) guides 284 that are provided in the retainer 282 and defines a relative rotational position of the retainer 282 with respect to the second shaft 182 when the pins 283 are in the forced free position. The rollers 281 may be balls or sprags.

A distance A in the radial direction between the outer peripheral surface portion of the second shaft 182 and the inner peripheral surface portions of the gears 184 and 186 is smaller than a diameter B of each of the rollers 281. Further, flat portions 182a are formed on an outer peripheral portion of the second shaft 182 at predetermined intervals in a circumferential direction, and on a center side in the circumferential direction of each of the flat portions 182a, the distance A is larger than the diameter B.

In other words, when the rollers 281 are held at center portions of the flat portions 182a in the circumferential direction, the rollers 281 do not mesh with the outer peripheral surface portion of the second shaft 182 and the inner peripheral surface portions of the gears 184 and 186 (non-engagement state), and relative rotation between the second shaft 182 and the gears 184 and 186 is allowed (forced free state).

On the other hand, when the rollers 281 are allowed to move in the circumferential direction relative to the second shaft 182, the rollers 281 mesh with the outer peripheral surface portion of the second shaft 182 and the inner peripheral surface portions of the gears 184 and 186 (engagement state), and the second shaft 182 and the gears 184 and 186 are connected in a manner of being rotatable integrally in two directions (forced free release state).

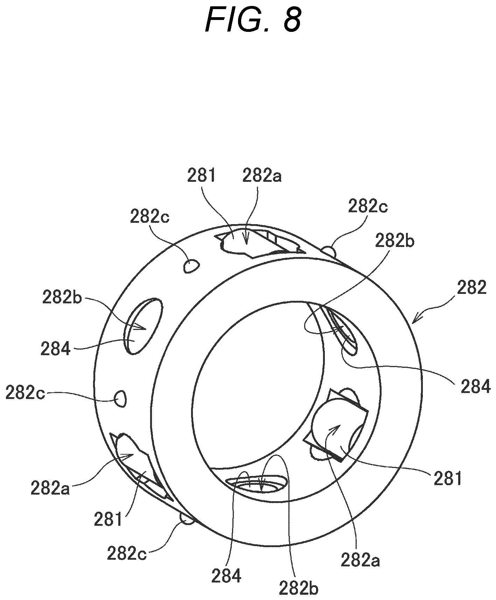

As shown in FIG. 8, the retainer 282 includes a plurality of roller holding portions 282a that are ring-shaped and capable of relatively rotating with respect to the second shaft 182 and the gears 184 and 186, and that hold the rollers 281, and a plurality of guide holding portions 282b that hold the guides 284.

In an outer peripheral surface of the retainer 282, a plurality of rubber balls 282c are embedded at predetermined intervals in the circumferential direction. These rubber balls 282c prevent unintended idling in the forced free release state by generating moderate friction between the gears 184 and 186 and the retainer 282. The members for generating friction between the gears 184 and 186 and the retainer 282 are not limited to the rubber balls 282c, and may be O-rings.

Returning to FIG. 7, each of the pins 283 includes a conical protrusion portion 283a on an outer end in the radial direction, and each of the guides 284 includes, on an inner end surface thereof in the radial direction, a conical recess portion 284a that fits to (engages with) the protrusion portion 283a. When the protrusion portion 283a of the pin 283 fits to the recess portion 284a of the guide 284, a relative rotation position of the retainer 282 with respect to the second shaft 182 is positioned at a predetermined position where the retainer 282 is in the forced free state by a guiding effect of the pins 283 and the guides 284.

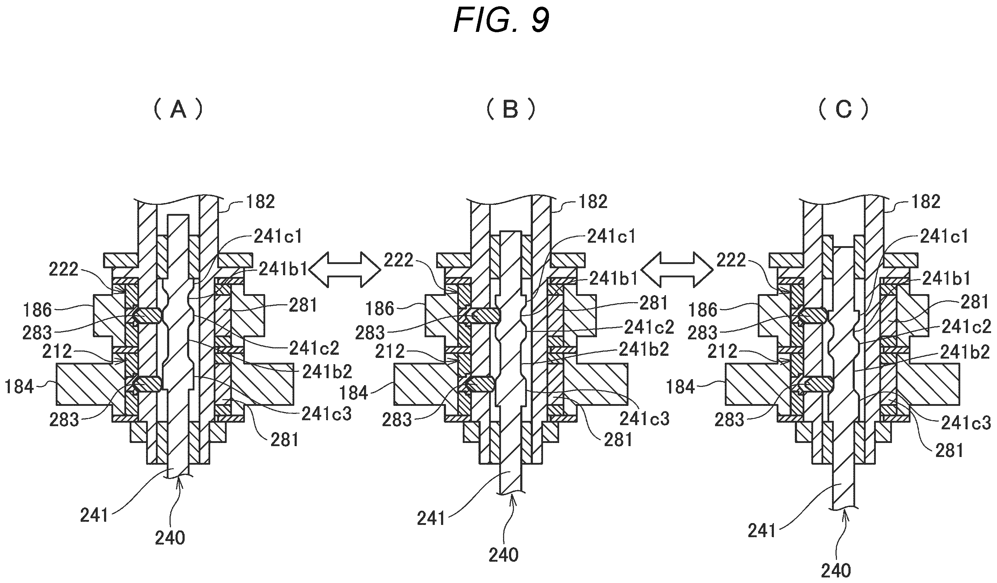

As shown in FIG. 9, the operation rod 241 includes, in the order from the upper side, a first large-diameter portion 241c1, a first small-diameter portion 241b1, a second large-diameter portion 241c2, a second small-diameter portion 241b2, and a third large-diameter portion 241c3 formed with predetermined lengths and intervals therebetween. The operation rod 241 is provided to be capable of simultaneously controlling the two connection and disconnection units 212 and 222, but may be provided separately for each of the connection and disconnection units 212 and 222.

In the following description, operation of the operation mechanism 240 that simultaneously controls the connection and disconnection units 212 and 222 will be described with reference to FIG. 9.

As shown in FIG. 9, the connection and disconnection units 212 and 222 are switched between the forced free state (hereinafter referred to as an off state as appropriate) and the forced free release state (hereinafter referred to as an on state as appropriate) by the operation mechanism 240.

When the operation rod 241 of the operation mechanism 240 is in an upper position shown in (A) of FIG. 9, the third large-diameter portion 241c3 pushes out the pin 283 of the connection and disconnection unit 222 in an outer diameter direction while the second large-diameter portion 241c2 pushes out the pin 283 of the connection and disconnection unit 212 in the outer diameter direction, thus setting the connection and disconnection unit 212 and the connection and disconnection unit 222 to the off state.

Further, when the operation rod 241 of the operation mechanism 240 is in a middle position shown in (B) of FIG. 9, the third large-diameter portion 241c3 pushes out the pin 283 of the connection and disconnection unit 212 in the outer diameter direction while the first small-diameter portion 241b1 allows the pin 283 of the connection and disconnection unit 222 to return in an inner diameter direction, thereby setting the connection and disconnection unit 222 to the on state and the connection and disconnection unit 212 to the off state.

Further, when the operation rod 241 of the operation mechanism 240 is in a lower position shown in (C) of FIG. 9, the second small-diameter portion 241b2 allows the pin 283 of the connection and disconnection unit 212 to return in the inner diameter direction while the first large-diameter portion 241c1 pushes out the pin 283 of the connection and disconnection unit 222 in the outer diameter direction, thus setting the connection and disconnection unit 222 to the off state and the connection and disconnection unit 212 to the on state.

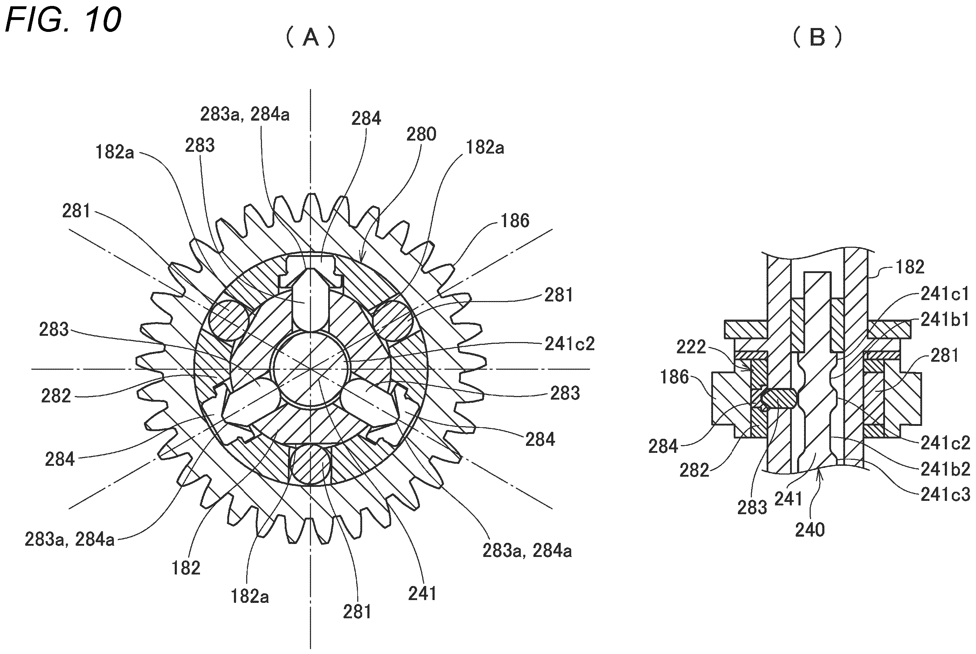

Next, the operation of the two-way clutch 280 will be described with reference to FIGs. 10 to 14, taking the connection and disconnection unit 222 as an example. In the following example, a case of a transition from (A) to (B) to (C) in FIG. 9 in the connection and disconnection unit 222 will be described as an example.

As shown in (A) and (B) of FIG. 10, in a state where the second large-diameter portion 241c2 of the operation rod 241 pushes out the pin 283 of the connection and disconnection unit 222 in the outer diameter direction, the protrusion portion 283a of the pin 283 fits to the recess portion 284a of the guide 284, and the relative rotational position of the retainer 282 with respect to the second shaft 182 is fixed at a predetermined position. In this state, since the rollers 281 are held at the center portion of the flat portion 182a in the circumferential direction, the rollers 281 do not mesh with the outer peripheral surface portion of the second shaft 182 and the inner peripheral surface portion of the second driven gear 186, and the state becomes the off state where the relative rotation between the second shaft 182 and the second driven gear 186 is allowed.

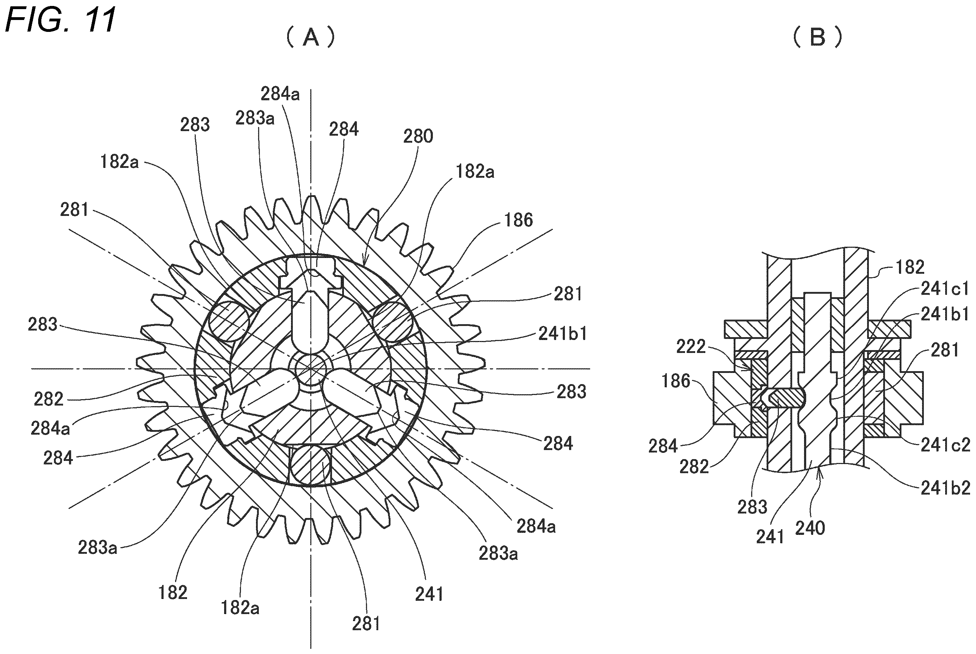

(A) and (B) of FIG. 11 show a state where the operation rod 241 moves from the position where the second large-diameter portion 241c2 pushes out the pin 283 of the connection and disconnection unit 222 in the outer diameter direction to the position where the first small-diameter portion 241b1 allows the pin 283 to return in the inner diameter direction. In FIG. 11, the pin 283 is moved in the inner diameter direction, but actually, at a timing when the relative rotation between the second shaft 182 and the second driven gear 186 occurs, the guides 284 of the retainer 282 that rotate together with the second driven gear 186 pushes the pin 283 back in the inner diameter direction on an inclined surface of the recess portion 284a.

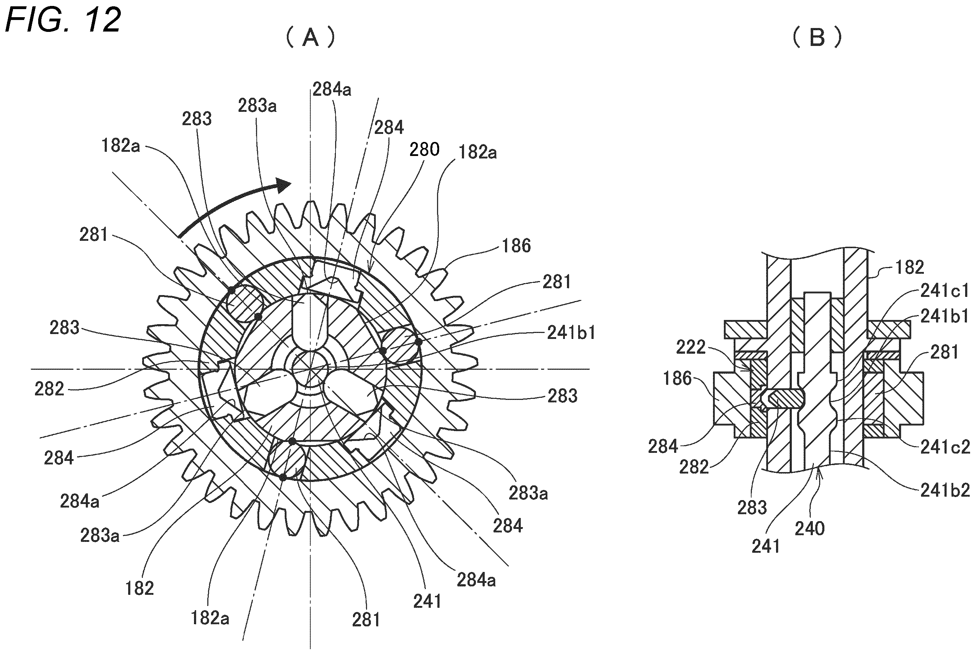

As shown in (A) and (B) of FIG. 12, in a state where the pin 283 is allowed to return in the inner diameter direction, when relative rotation occurs between the second shaft 182 and the second driven gear 186 in a normal rotation direction shown by an arrow in the drawing, the retainer 282 that rotates together with the second driven gear 186 moves the rollers 281 in the normal rotation direction with respect to the second shaft 182. Accordingly, the rollers 281 mesh with the outer peripheral surface portion of the second shaft 182 and the inner peripheral surface portion of the second driven gear 186, and a normal-rotation on state is created in which the second shaft 182 and the second driven gear 186 are rotated integrally in the normal rotation direction.

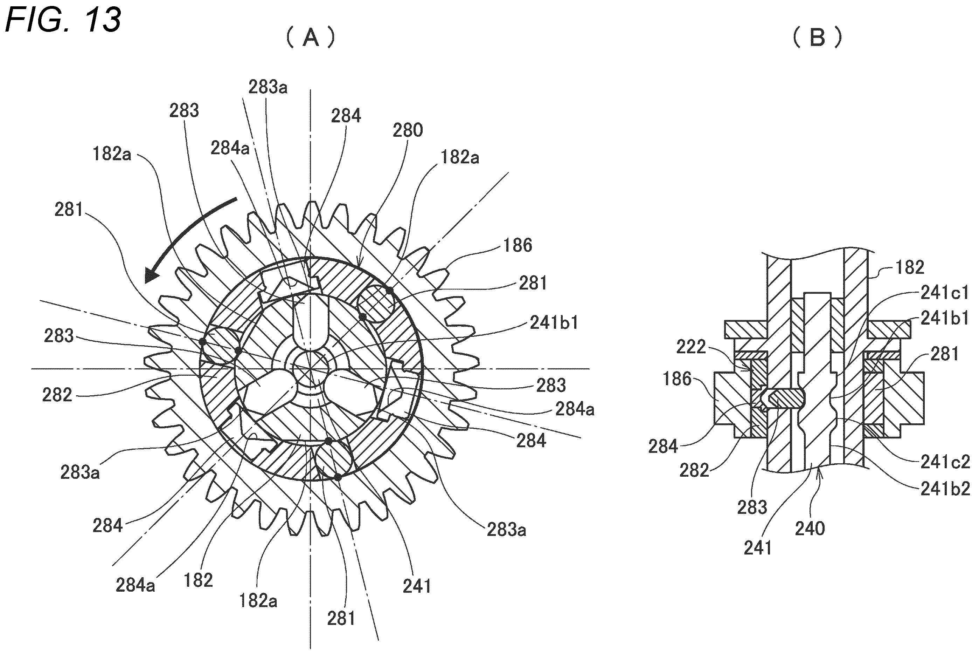

As shown in (A) and (B) of FIG. 13, in a state where the pin 283 is allowed to return in the inner diameter direction, when relative rotation occurs between the second shaft 182 and the second driven gear 186 in a reverse rotation direction shown by an arrow in the drawing, the retainer 282 that rotates together with the second driven gear 186 moves the rollers 281 in the reverse rotation direction with respect to the second shaft 182. Accordingly, the rollers 281 mesh with the outer peripheral surface portion of the second shaft 182 and the inner peripheral surface portion of the second driven gear 186, and a reverse-rotation on state is created in which the second shaft 182 and the second driven gear 186 are rotated integrally in the reverse rotation direction.

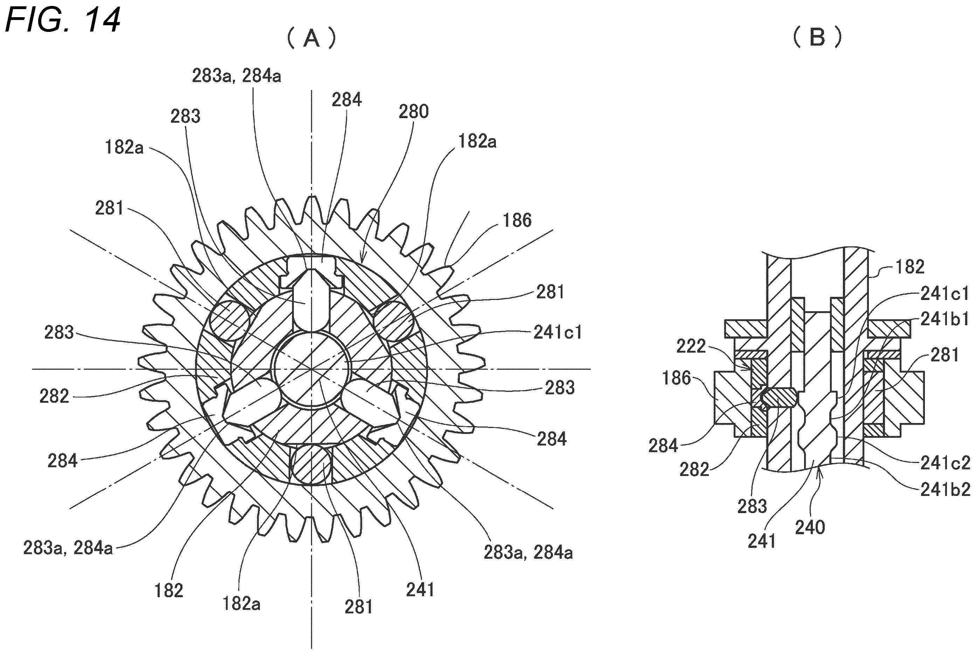

As shown in (A) and (B) of FIG. 14, when the operation rod 241 moves from a position where the first small-diameter portion 241b1 allows the pin 283 of the connection and disconnection unit 222 to return in the inner diameter direction, to a position where the first large-diameter portion 241c1 pushes out the pin 283 in the outer diameter direction, the protrusion portion 283a of the pin 283 fits to the recess portion 284a of the guide 284, and the relative rotation position of the retainer 282 with respect to the second shaft 182 is fixed at a predetermined position, by the guiding effect of the pin 283 and the guide 284. In this state, since the rollers 281 are held at the center portion of the flat portion 182a in the circumferential direction, the rollers 281 do not mesh with the outer peripheral surface portion of the second shaft 182 and the inner peripheral surface portion of the second driven gear 186, and the state becomes the off state where the relative rotation between the second shaft 182 and the second driven gear 186 is allowed.

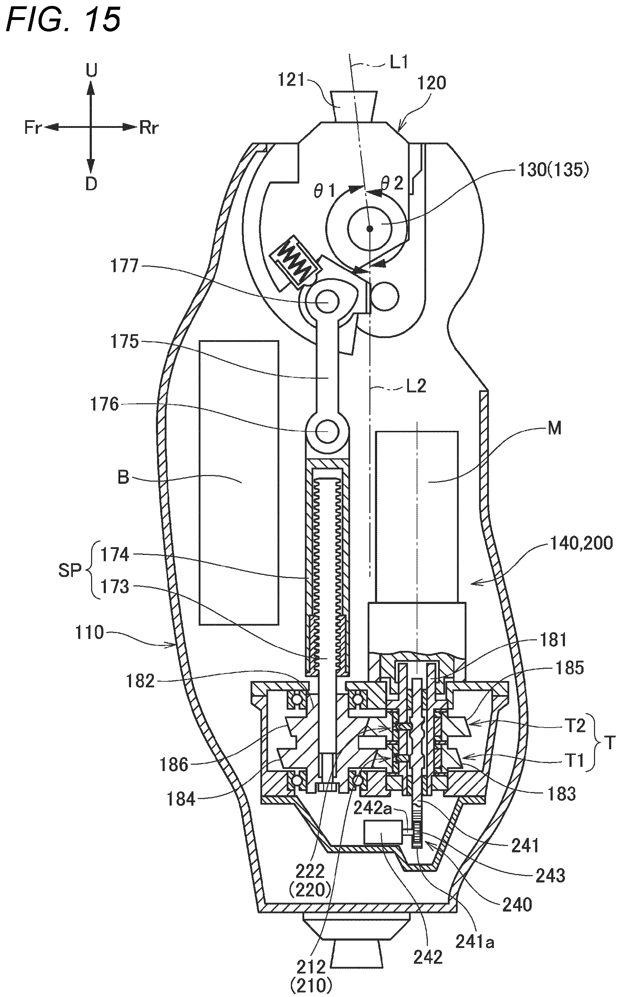

In the above embodiment, the connection and disconnection units 212 and 222 and the operation mechanism 240 are provided on a second shaft 182 side, but may be provided on a first shaft 181 side as in a second embodiment shown in FIG. 15. That is, in the electric prosthetic leg 1 of the second embodiment, the connection and disconnection unit 212 of the first connection and disconnection mechanism 210 is provided between the first drive gear 183 and the first shaft 181, and the connection and disconnection unit 222 of the second connection and disconnection mechanism 220 is provided between the second drive gear 185 and the first shaft 181. Since other configurations are substantially the same as or similar to those of the first embodiment, the following description will be made by taking the electric prosthetic leg 1 of the first embodiment as an example.

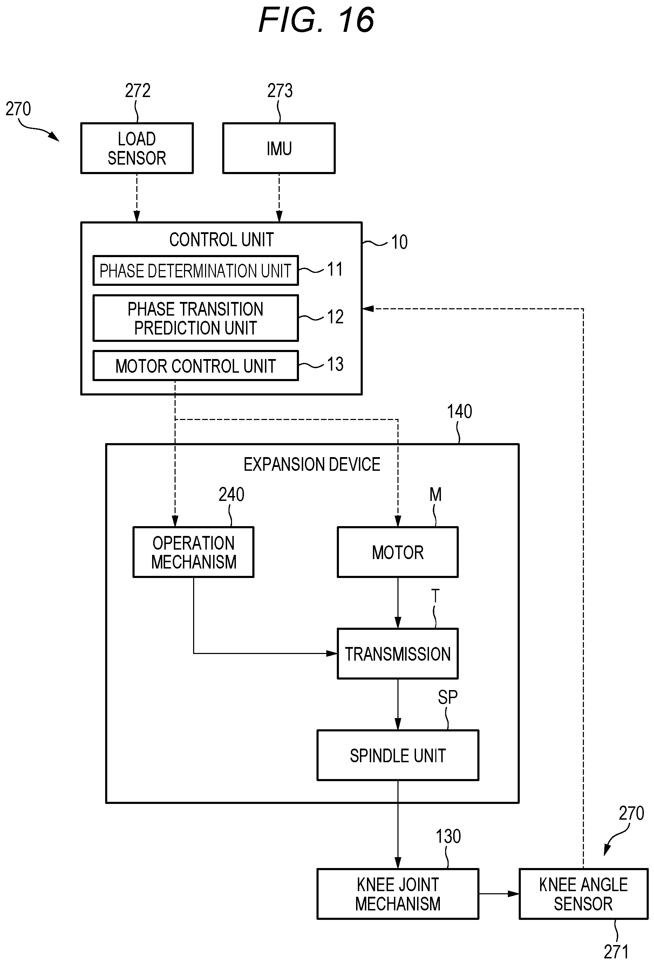

As shown in FIG. 16, the sensor device 270 includes a knee angle sensor 271 provided in the knee joint mechanism 130 (coupling axis 135), a load sensor 272 incorporated in the adapter 122, and an inertial measurement unit (IMU) 273 mounted on a substrate (not shown) disposed in the vicinity of the motor M.

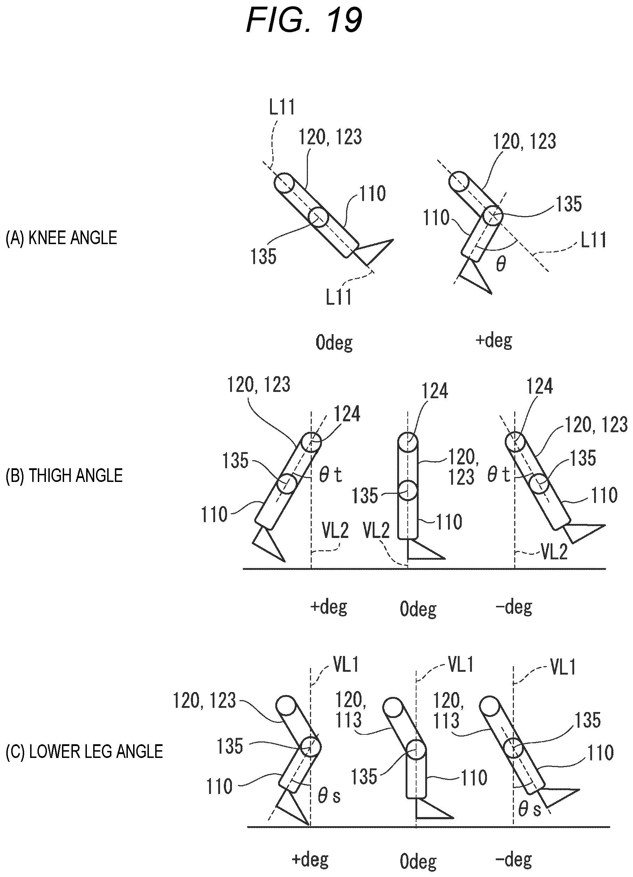

The knee angle sensor 271 detects a knee angle (θ [deg]). As shown in (A) of FIG. 19, the knee angle (θ [deg]) is a formed angle formed by an extension line L11 of the above-knee member 120 passing through the coupling axis 135 and the below-knee member 110. In other words, the knee angle θ is an angle obtained by subtracting the second formed angle θ2 from 180 [deg], and is a supplementary angle of the second formed angle θ2. The knee angle θ takes a negative value when the below-knee member 110 is in front of the extension line L11, and the knee angle θ takes a positive value (+deg) when the below-knee member 110 is behind the extension line L11. Therefore, a possible range of the second formed angle θ2 is about 60 [deg] to 185 [deg], and a possible range of the knee angle θ is about -5 [deg] to 120 [deg].

The load sensor 272 detects a weight which the electric prosthetic leg 1 receives, in other words, a load from the user of the electric prosthetic leg 1. The load sensor 272 is set such a tensile load leads to a positive value and a compression load leads to a negative value. Therefore, in a weighted state where the electric prosthetic leg 1 is in contact with the ground (hereinafter, may be referred to as a stance phase), a negative value is obtained when a weight (compression load) is applied to the electric prosthetic leg 1 from the outside, and in a non-weighted state where the electric prosthetic leg 1 is separated from the ground (hereinafter, may be referred to as a swing phase), a positive value is obtained when a tensile load is applied due to an own weight of the electric prosthetic leg 1 although no weight is received from the outside.

The IMU 273 acquires angular velocities in three axes and accelerations in the three axes. When three axes of an orthogonal coordinate system are an X-axis, a Y-axis, and a Z-axis, the IMU 273 detects an X-axis angular velocity ωx [deg/s], a Y-axis angular velocity ωy [deg/s], a Z-axis angular velocity ωz [deg/s], an X-axis acceleration Ax [m/s²], a Y-axis acceleration Ay [m/s²], and a Z-axis acceleration Az [m/s²].

Next, the control unit 10 that controls the electric prosthetic leg 1 will be described.

The control unit 10 drives the electric prosthetic leg 1 in a stair ascending mode to be described later when the user walks up stairs, and drives the electric prosthetic leg 1 in a flat ground and stair descending mode to be described later when the user walks to advance on a flat ground or walks to ascend stairs. The control unit 10 receives information from the sensor device 270 and controls the electric prosthetic leg 1 in each mode. More specifically, the control unit 10 receives information from the knee angle sensor 271, the load sensor 272, and the IMU 273. The control unit 10 acquires the knee angle θ from the knee angle sensor 271, acquires the load from the load sensor 272, and calculates a lower leg angle θs from the angular velocities in the three axes and the accelerations in the three axes detected by the IMU 273. The control unit 10 calculates a thigh angle θt from the knee angle θ and the lower leg angle θs. In addition, the control unit 10 can calculate an angular velocity or an angular acceleration of each of the knee angle θ, the lower leg angle θs, and the thigh angle θt, accelerations of the below-knee member 110 and the above-knee member 120, and the like.

Here, as shown in (C) of FIG. 19, the lower leg angle θs is a formed angle formed between a center line extending in an extending direction of the below-knee member 110 and centered on the coupling axis 135 and a vertical line VL1 passing through the coupling axis 135. When the below-knee member 110 is in front of the vertical line VL1, the lower leg angle θs takes a negative value (-deg), and when the below-knee member 110 is behind the vertical line VL1, the lower leg angle θs takes a positive value (+deg).

Further, as shown in (B) of FIG. 19, the thigh angle θt is a formed angle formed by a vertical line VL2 passing through a hip joint 124 of a thigh 123 to which the above-knee member 120 is attached, and a center line extending in an extending direction of the above-knee member 120. When the above-knee member 120 is in front of the vertical line VL2, the thigh angle θt takes a negative value (-deg), and when the above-knee member 120 is behind the vertical line VL2, the thigh angle θt takes a positive value (+deg).

In each mode, the control unit 10 controls the operation mechanism 240 (the servo motor 242) that switches between the disconnection state and the power transmittable state of the first connection and disconnection mechanism 210 and the second connection and disconnection mechanism 220 based on the information, and controls the motor M that outputs power for stretching or bending the electric prosthetic leg 1.

Referring back to FIG. 16, the control unit 10 includes a phase determination unit 11 that determines whether the electric prosthetic leg 1 is in the stance phase or the swing phase, a phase transition prediction unit 12 that predicts a transition from the stance phase to the swing phase and a transition from the swing phase to the stance phase, and a motor control unit 13 that controls the motor M that expands and contracts the expansion-contraction device 140 and the servo motor 242 that drives the operation rod 241. The phase determination unit 11 functions as a weight transition acquisition unit that acquires that the electric prosthetic leg 1 becomes the weighted state (stance phase) or the non-weighted state (swing phase), and the phase transition prediction unit 12 functions as a weight acquisition unit that acquires a weight which the electric prosthetic leg 1 receives, or acquires the weighted state (stance phase) or the non-weighted state (swing phase).

When the load detected by the load sensor 272 is equal to or larger than a first threshold (for example, -90 [N]), the phase determination unit 11 determines the swing phase, and when the load is equal to or less than a second threshold (for example, -110 [N]), the phase determination unit 11 determines the stance phase. Further, the phase determination unit 11 determines that the phase transitions from the stance phase to the swing phase when the load becomes equal to or larger than the first threshold (for example, -90 [N]) in a state where the stance phase is determined. On the other hand, when the load becomes equal to or less than the second threshold (for example, -110 [N]) in a state where the swing phase is determined, the transition from the swing phase to the stance phase is determined. The first threshold and the second threshold may be the same value, but are preferably different values by providing hysteresis. This can further prevent hunting.

The phase transition prediction unit 12 predicts the transition from the swing phase to the stance phase and a transition from the stance phase to the swing phase to acquire the transition, based on at least one of the knee angle θ, the lower leg angle θs, an angular velocity ωs of the lower leg angle θs (hereinafter, a lower leg angular velocity ωs), and the thigh angle θt. By the phase transition prediction unit 12 predicting a state transition during the walking, a terminal impact and/or trip to be described later can be prevented.

The motor control unit 13 controls the motor M to extend and contract the expansion-contraction device 140 so as to stretch or bend the electric prosthetic leg 1. Further, the motor control unit 13 controls the servo motor 242 to move the operation rod 241, so as to switch the first connection and disconnection mechanism 210 and the second connection and disconnection mechanism 220 between the disconnection state and the power transmittable state. Further, in the power transmittable state, a speed change state (hereinafter, referred to as a high-torque-side connection state) where the power transmission state is established via the first transmission mechanism T1 and a speed change state (hereinafter, referred to as a high-speed-rotation-side connection state) where the power transmission state is established via the second transmission mechanism T2 are switched.

### <Stair Ascending Mode>

In the electric prosthetic leg 1 configured in this way, it is possible to smoothly ascend stairs, which has been required to be done one by one by a leg on a non-prosthetic leg side (healthy leg), with a passive prosthetic leg including a passive damper in the related art. A mode in which the user walks upstairs is the stair ascending mode.

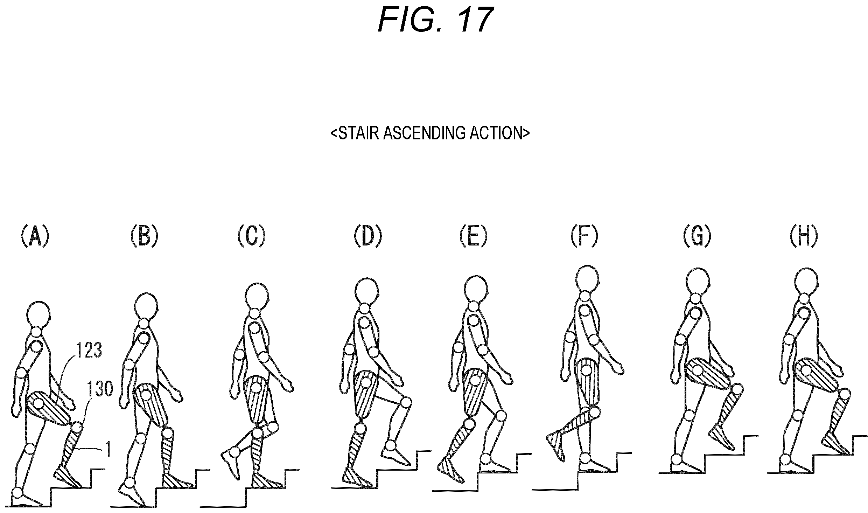

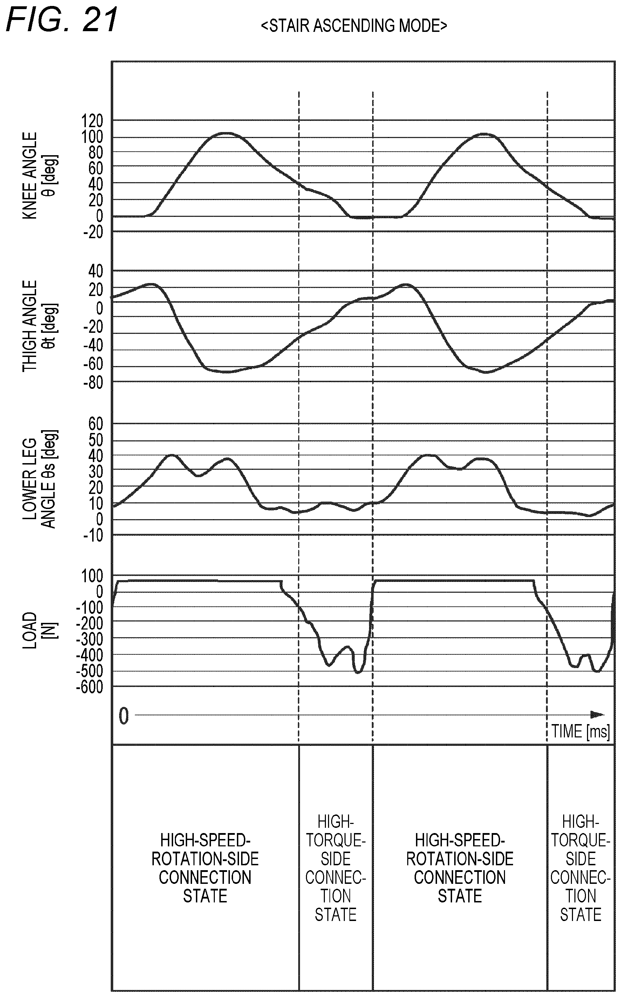

FIG. 17 is a diagram showing actions (stair ascending actions) of a human and the electric prosthetic leg when ascending stairs, and FIG. 21 is a graph showing a relationship of the knee angle θ, the thigh angle θt, the lower leg angle θs, and the load in the stair ascending mode.

(A) to (D) of FIG. 17 are diagrams showing the stance phase, (D) and (E) of FIG. 17 are diagrams showing a transition phase from a stance to an initial swing, (E) to (G) of FIG. 17 are diagrams showing an initial swing phase, (G) of FIG. 17 is a diagram showing a transition phase from the initial swing to a terminal swing and a terminal swing phase, and (H) of FIG. 17 is a diagram showing a transition phase from the terminal swing to the stance and the stance phase.

As shown in (A) to (D) of FIG. 17, large power is required when the knee joint mechanism 130 is stretched from a bending state, in a state where a load is applied to the electric prosthetic leg 1 when moving the electric prosthetic leg 1 forward to ascending stairs.

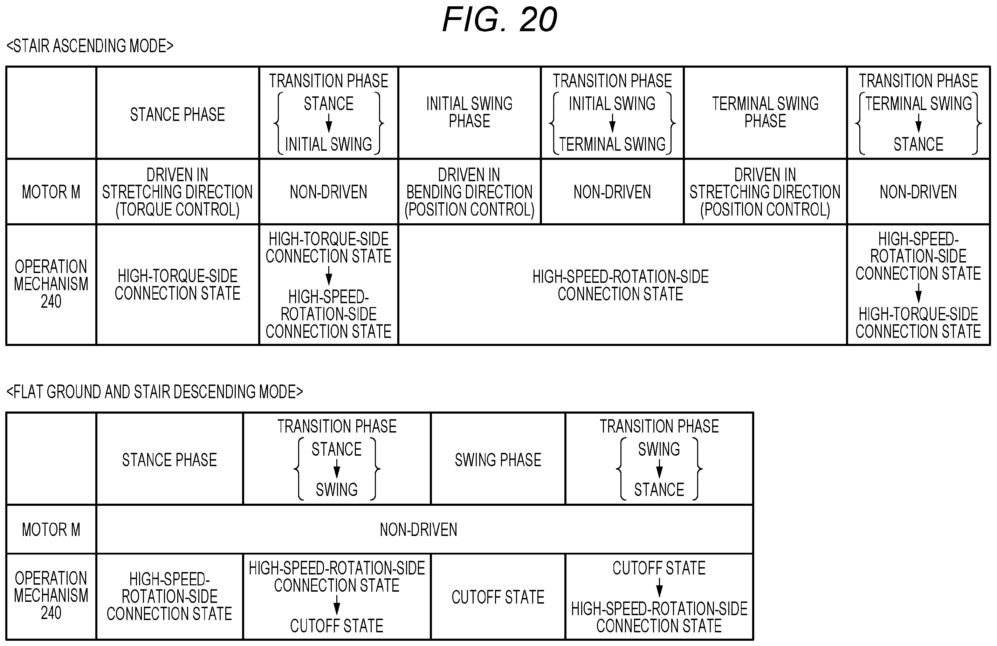

During the stance ((A) to (D) of FIG. 17), the motor control unit 13 drives the servo motor 242 to set the first connection and disconnection mechanism 210 and the second connection and disconnection mechanism 220 into a high-torque-side connection state (a stance phase in FIG. 20). In this high-torque-side connection state, when the motor M is rotated in a stretching direction, that is, in a direction of enlarging the second formed angle θ2, the power of the motor M is transmitted to the first shaft 181, the first drive gear 183, the first driven gear 184, the connection and disconnection unit 212 of the first connection and disconnection mechanism 210, the second shaft 182, and the spindle unit SP. Accordingly, the sleeve 174 is expanded to be separated from the transmission T, and the above-knee member 120 to which the sleeve 174 is coupled is rotated about the coupling axis 135 with respect to the below-knee member 110 to which the transmission T is attached, and the knee joint mechanism 130 is stretched. Since the power for the stretching is power whose torque is increased during deceleration by the first transmission mechanism T1, even when a large load is applied to the electric prosthetic leg 1 when moving the electric prosthetic leg 1 forward to ascend stairs, the knee joint mechanism 130 can also be reliably stretched from the bending state.

On the other hand, in order to smoothly ascend stairs, as shown in (D) to (G) of FIG. 17, it is necessary to bend the knee joint mechanism 130 from the stretched state while a load is applied to the healthy leg. At this time, it is necessary to quickly fold the electric prosthetic leg 1 and then quickly land the electric prosthetic leg 1 on a next stair. When the knee joint mechanism 130 is bent from the stretched state, large power is not required, but quick action is required.

Specifically, during the transition from the stance to the initial swing ((D) to (E) of FIG. 17), the motor control unit 13 drives the servo motor 242 to cause the first connection and disconnection mechanism 210 and the second connection and disconnection mechanism 220 to transition from the high-torque-side connection state to the high-speed-rotation-side connection state (a transition phase (from the stance state to the initial swing) in FIG. 20), and during this time, the motor M is set into the non-driven state. The non-driven state of the motor M means that the motor M is controlled to generate no power or to be stopped. In the initial swing thereafter (the initial swing phase in FIG. 20), the motor M is rotated in a bending direction opposite to the stretching direction in the high-speed-rotation-side connection state ((E) to (G) of FIG. 17). Then, the power of the motor M is transmitted to the first shaft 181, the second drive gear 185, the second driven gear 186, the connection and disconnection unit 222 of the second connection and disconnection mechanism 220, the second shaft 182, and the spindle unit SP. Accordingly, the sleeve 174 is contracted to approach the transmission T, and the below-knee member 110 to which the transmission T is attached is rotated about the coupling axis 135 with respect to the above-knee member 120 to which the sleeve 174 is coupled, and the knee joint mechanism 130 is bent.

During the transition from the initial swing to the terminal swing ((G) of FIG. 17), the motor M is once set into the non-driven state (a transition phase in FIG. 20 (from the initial swing to the terminal swing)). Then, during the terminal swing ((G) to (H) of FIG. 17), the motor M is rotated in the stretching direction (the terminal swing phase in FIG. 20) in the high-speed-rotation-side connection state. Then, the power of the motor M is transmitted to the first shaft 181, the first drive gear 183, the first driven gear 184, the connection and disconnection unit 222 of the first connection and disconnection mechanism 210, the second shaft 182, and the spindle unit SP. Accordingly, the sleeve 174 is expanded to be separated from the transmission T, and the above-knee member 120 to which the sleeve 174 is coupled is rotated about the coupling axis 135, and the knee joint mechanism 130 is stretched. Since the power for the bending and the stretching during the swing is power whose torque is reduced during acceleration by the second transmission mechanism T2, the knee joint mechanism 130 can be quickly bent and stretched.

During the transition from the terminal swing to the stance ((H) in FIG. 17), the motor control unit 13 drives the servo motor 242 to cause the first connection and disconnection mechanism 210 and the second connection and disconnection mechanism 220 to transition from the high-speed-rotation-side connection state to the high-torque-side connection state (a transition phase (from the terminal swing to the stance) in FIG. 20), and sets the motor M into the non-driven state.

In the stair ascending mode, when the motor control unit 13 controls the motor M, the control is performed based on a torque target value which is a target value of a torque related to a torque for the expansion-contraction device 140 to enlarge or reduce the formed angle when the first connection and disconnection mechanism 210 and the second connection and disconnection mechanism 220 are in the high-torque-side connection state. On the other hand, the motor control unit 13 controls based on a position target value which is a target value of a position related to the formed angle enlarged or reduced by the expansion-contraction device 140 when the first connection and disconnection mechanism 210 and the second connection and disconnection mechanism 220 are in the high-speed-rotation-side connection state.

### <Flat Ground and Stair Descending Mode>

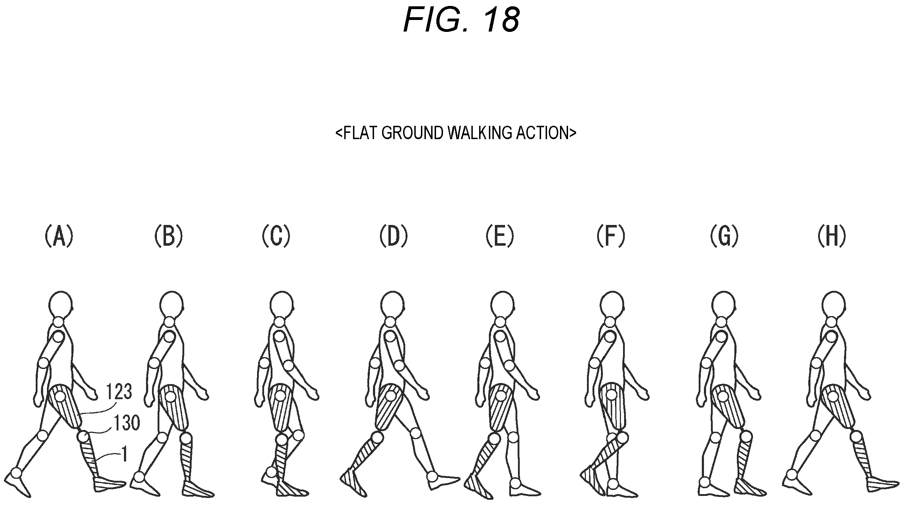

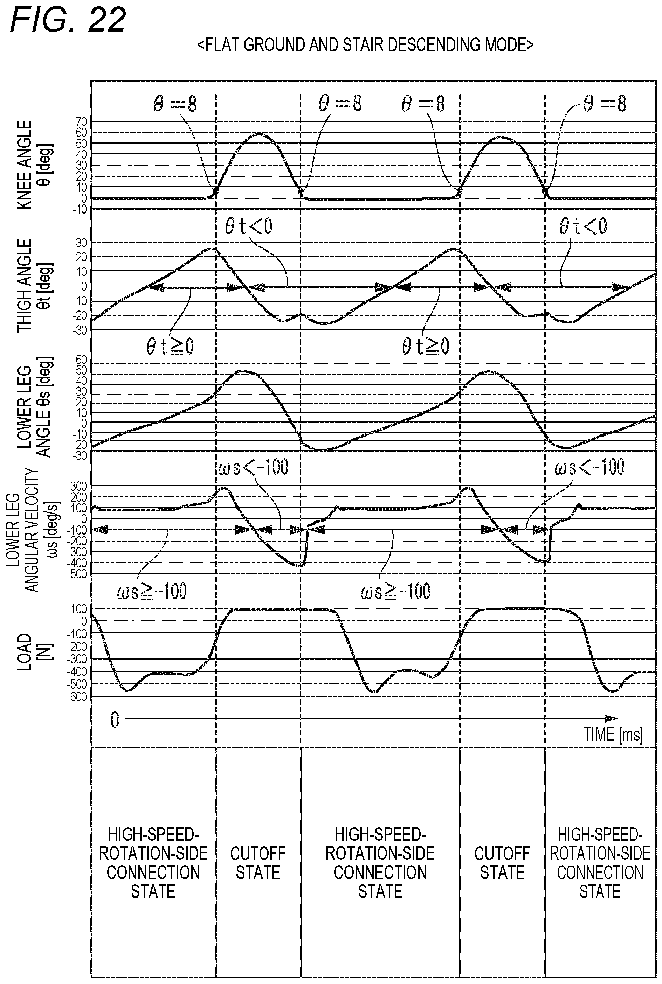

A mode when the user descends stairs or walks on a flat ground is the flat ground and stair descending mode. The flat ground means that there is no step such as a stair, and is a concept including an uphill slope and a downhill slope in addition to a horizontal place. FIG. 18 is a diagram showing actions (flat ground walking actions) of the human and the electric prosthetic leg when walking on the flat ground, and FIG. 22 is a graph showing a relationship of the knee angle θ, the thigh angle θt, the lower leg angle θs, and the load in the flat ground and stair descending mode.

(A) to (D) of FIG. 18 are diagrams showing the stance phase, (D) of FIG. 18 is a diagram showing the transition phase from the stance to the swing, (E) to (H) of FIG. 18 are diagrams showing the swing phase, and (H) of FIG. 18 is a diagram showing the transition phase from the swing to the stance.

In the flat ground and stair descending mode, the motor M is normally in the non-driven state. In the non-driven state of the motor M, as shown in (A) to (D) of FIG. 18, it is necessary to prevent so-called giving way in the stance phase in which a load is applied to the electric prosthetic leg 1.

During the stance ((A) to (D) of FIG. 18), the motor control unit 13 drives the servo motor 242 to set the first connection and disconnection mechanism 210 and the second connection and disconnection mechanism 220 into the high-speed-rotation-side connection state. (Stance Phase of FIG. 20). In the high-speed-rotation-side connection state, since the connection and disconnection unit 222 is in the on state, the motor M and the spindle unit SP are in the power transmission state via the second transmission mechanism T2. In the high-speed-rotation-side connection state, when the motor M is held in the non-driven state, an external force in the bending direction acting on the electric prosthetic leg 1 is transmitted from the spindle unit SP to the motor M via the second transmission mechanism T2, frictions of the motor M and the transmission T are utilized to dampen the external force in the bending direction, thus preventing the so-called giving way.

On the other hand, as shown in (E) to (H) of FIG. 18, it is necessary to freely swing the electric prosthetic leg 1 during the swing in which no weight from the outside is applied to the electric prosthetic leg 1. Therefore, during the transition from the stance to the swing ((D) of FIG. 18), the motor control unit 13 drives the servo motor 242 to set the first connection and disconnection mechanism 210 and the second connection and disconnection mechanism 220 into the disconnection state (transition phase (from the stance to the swing) in FIG. 20). In the disconnection state, since the connection and disconnection unit 212 and the connection and disconnection unit 222 are in the off state, the motor M and the spindle unit SP are in the disconnection state of being not connected with each other, during the swing (the swing phase in FIG. 20). In this state, when the motor M is held in the non-driven state, free stretching and bending of the knee joint mechanism 130 are allowed. Descending stairs is controlled in the same way as walking on flat ground.

In the transition from the swing to the stance ((H) in FIG. 18), the motor control unit 13 drives the servo motor 242 to cause the first connection and disconnection mechanism 210 and the second connection and disconnection mechanism 220 to transition from the disconnection state to the high-speed-rotation-side connection state (the transition phase (from the swing to the stance) in FIG. 20).

As shown in FIG. 20, the control unit 10 controls the first connection and disconnection mechanism 210 and the second connection and disconnection mechanism 220 to the high-torque-side connection state where the first connection and disconnection mechanism 210 is connected in the stance phase in the stair ascending mode, and controls the first connection and disconnection mechanism 210 and the second connection and disconnection mechanism 220 to the high-speed-rotation-side connection state where the second connection and disconnection mechanism 220 is connected in the swing phase (the initial swing phase, the transition phase (from the initial swing phase to the terminal swing phase), and the terminal swing phase) in the stair ascending mode. Further, the control unit 10 controls the first connection and disconnection mechanism 210 and the second connection and disconnection mechanism 220 to the high-speed-rotation-side connection state where the second connection and disconnection mechanism 220 is connected in the stance phase in the flat ground and stair descending mode, and to the disconnection state where the first connection and disconnection mechanism 210 and the second connection and disconnection mechanism 220 are disconnected in the swing phase in the flat ground and stair descending mode. The reason why the high-speed-rotation-side connection state is set during the stance phase in the flat ground and stair descending mode is to prevent the giving way, and the high-torque-side connection state may be set without necessarily requiring the high-speed-rotation-side connection state.

That is, when summarizing without separating the stair ascending mode and the flat ground and stair descending mode, the control unit 10 controls the first connection and disconnection mechanism 210 and the second connection and disconnection mechanism 220 to a first connection state where any one of the first connection and disconnection mechanism 210 and the second connection and disconnection mechanism 220 is connected in the stance phase which is the weighted state, and controls the first connection and disconnection mechanism 210 and the second connection and disconnection mechanism 220 to the disconnection state where the first connection and disconnection mechanism 210 and the second connection and disconnection mechanism 220 are disconnected or to a second connection state where the other of the first connection and disconnection mechanism 210 and the second connection and disconnection mechanism 220 is connected, in the swing phase which is the non-weighted state. By appropriately switching the states of the first connection and disconnection mechanism 210 and the second connection and disconnection mechanism 220 between the stance phase in which the electric prosthetic leg 1 receives a weight applied from the outside and the swing phase in which no weight is received, convenience of the electric prosthetic leg 1 is improved.

In the stair ascending mode, the control unit 10 controls to switch from the high-torque-side connection state to the high-speed-rotation-side connection state when the phase transition prediction unit 12 acquires the transition from the stance phase to the swing phase, and controls to switch from the high-speed-rotation-side connection state to the high-torque-side connection state when the phase transition prediction unit 12 acquires the transition from the swing phase to the stance phase.

In the flat ground and stair descending mode, the control unit 10 controls to switch from the high-speed-rotation-side connection state to the disconnection state when the phase transition prediction unit 12 acquires the transition from the stance phase to the swing phase, and controls to switch from the disconnection state to the high-speed-rotation-side connection state when the phase transition prediction unit 12 acquires the transition from the swing phase to the stance phase.

### (Terminal Impact Prevention Control)

During the swing phase in the flat ground and stair descending mode, since the first connection and disconnection mechanism 210 and the second connection and disconnection mechanism 220 are controlled to the disconnection state, the knee joint mechanism 130 is allowed to rotate freely, and a terminal impact may occur. The terminal impact is a collision sound generated by a collision of the first contact portion 152, which is a portion of the above-knee member 120 that prevents the electric prosthetic leg 1 from excessively stretching in the terminal swing in the walking action, and the stopper member 151 which is a portion of the below-knee member 110 (see FIG. 3).

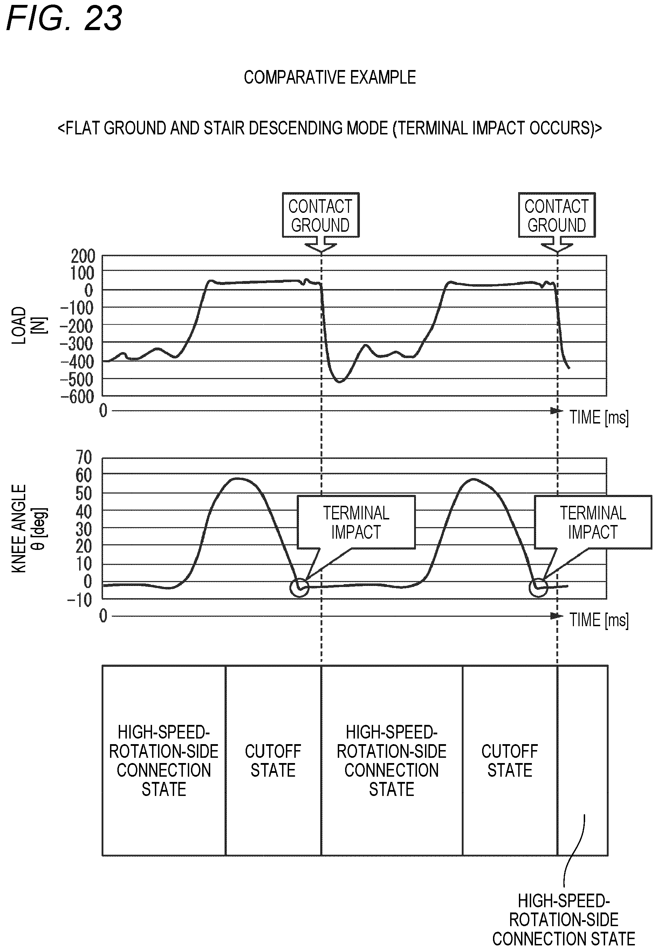

As shown in a comparative example of FIG. 23, when the electric prosthetic leg 1 contacts the ground during walking on the flat ground, that is, when transitioning from the swing phase to the stance phase, the second formed angle θ2 (knee-back angle) is substantially a maximum formed angle (185 [deg]), in other words, the knee angle θ becomes substantially a minimum formed angle (-5 [deg]), but the terminal impact may occur at this time.

As shown in the comparative example of FIG. 23, when the phase determination unit 11 determines the transition from the swing phase to the stance phase when the electric prosthetic leg 1 contacts the ground, and the state is switched to the connection state after the determination, the terminal impact may occur. The occurrence of the terminal impact impairs the convenience of the electric prosthetic leg 1.

In order to prevent the occurrence of the terminal impact, it is necessary to control the first connection and disconnection mechanism 210 and the second connection and disconnection mechanism 220 to the power transmission state before the transition from the swing phase to the stance phase.

Therefore, the phase transition prediction unit 12 predicts the transition of the electric prosthetic leg 1 from the swing phase to the stance phase to acquire the transition, and the motor control unit 13 controls to switch the first connection and disconnection mechanism 210 and the second connection and disconnection mechanism 220 from the disconnection state to the high-speed-rotation-side connection state before the electric prosthetic leg 1 starts to transition from the swing phase to the stance phase.

Specifically, in a case where the electric prosthetic leg 1 is in the swing phase and the control unit 10 controls the first connection and disconnection mechanism 210 and the second connection and disconnection mechanism 220 to the disconnection state, the motor control unit 13 controls to switch the first connection and disconnection mechanism 210 and the second connection and disconnection mechanism 220 from the disconnection state to the high-speed-rotation-side connection state when the following condition (P1) is satisfied, preferably at least one of (P2) and (P3) is further satisfied.

### (P1)

The phase transition prediction unit 12 detects that the second formed angle θ2 is equal to or larger than a threshold smaller than the maximum formed angle by a predetermined angle, in other words, the knee angle θ is equal to or smaller than a threshold.

### (P2)

The thigh angle θt is within a first predetermined range.

### (P3)

The lower leg angular velocity ωs is in a second predetermined range.

(P1) is a condition for determining a time immediately before the occurrence of the terminal impact, and (P2) and (P3) are conditions for recognizing the flat ground walking action (the swing phase of the electric prosthetic leg 1). Although the conditions (P2) and/or (P3) are not necessarily required, the terminal impact can be more appropriately prevented by using these conditions as determination conditions.

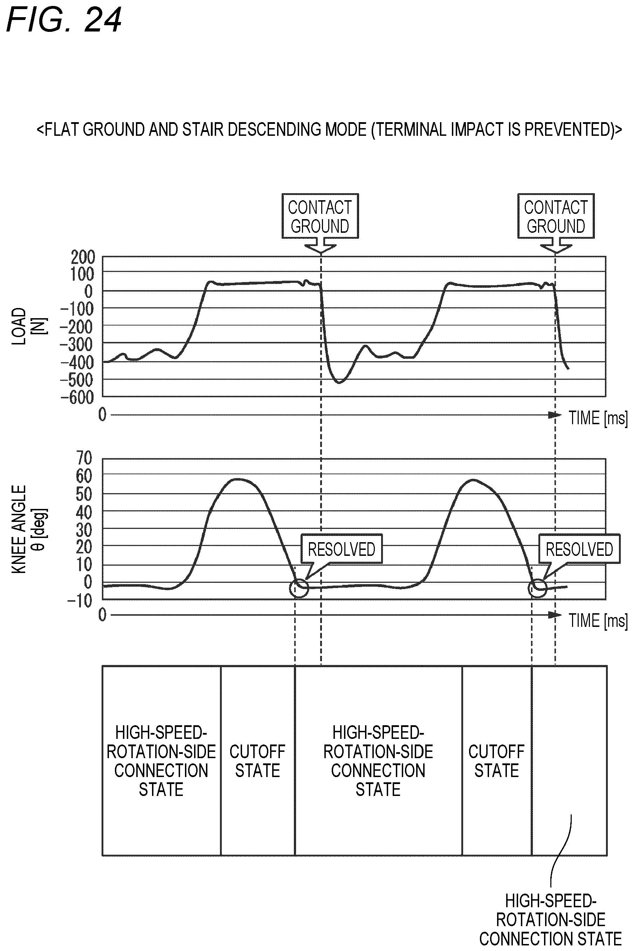

The threshold of the second formed angle θ2 is, for example, 172 [deg]. In other words, the knee angle θ is 8 [deg]. When the second formed angle θ2 is equal to or larger than 172 [deg], in other words, when the knee angle θ is 8 [deg] or less, the occurrence of the terminal impact can be predicted, as shown in FIGs. 22 and 24.

The first predetermined range is, for example, a negative range. That is, as described above, since the thigh angle θt takes a negative value when the above-knee member 120 is in front of the vertical line VL2 (see (B) of FIG. 19), this condition makes it possible to determine that the electric prosthetic leg 1 is swung forward in flat ground walking action, that is, in the swing phase in the flat ground walking action (see FIG. 22).

The second predetermined range is, for example, a range of -100 [deg/s] or less. That is, as described above, since the lower leg angle θs takes a negative value when the below-knee member 110 is in front of the vertical line VL1 (see (C) of FIG. 19), the lower leg angular velocity ωs being in the range of -100 [deg/s] or less means that a speed of swinging the below-knee member 110 forward is high. Since the speed of swinging the below-knee member 110 forward is high, it is possible to determine the swing phase in the flat ground walking action (see FIG. 22).

From the graph of FIG. 22 showing a relationship of the knee angle θ, the thigh angle θt, the lower leg angle θs, and the load in the flat ground and stair descending mode, it can be seen that the thigh angle θt is in a negative range and the lower leg angular velocity ωs is -100 [deg/s] or less, immediately before the occurrence of the terminal impact.

Therefore, when the thigh angle θt is in a negative range and the lower leg angular velocity ωs is -100 [deg/s] or less at a time before a time when the knee angle θ becomes substantially the minimum formed angle (the second formed angle θ2 (knee-back angle) is substantially the maximum formed angle of 185 degrees), and the phase transition prediction unit 12 detects that the knee angle θ is 8 [deg] or less (the second formed angle θ2 is equal to or larger than 172 [deg]), the control unit 10 predicts the transition of the electric prosthetic leg 1 from the swing phase to the stance phase, and controls to switch from the disconnection state to the high-speed-rotation-side connection state.

When the first connection and disconnection mechanism 210 and the second connection and disconnection mechanism 220 are in the high-speed-rotation-side connection state, since an external force in the stretching direction acting on the electric prosthetic leg 1 is transmitted from the spindle unit SP to the motor M via the second transmission mechanism T2, friction of the motor M and the transmission T can be used to dampen the external force in the stretching direction, and the terminal impact is prevented.

Further, the control unit 10 controls to maintain the high-speed-rotation-side connection state for a predetermined time (for example, 300 [ms]) after switching from the disconnection state to the high-speed-rotation-side connection state. Then, when the phase determination unit 11 acquires, during the predetermined time, that the electric prosthetic leg 1 reaches the stance phase, the control unit 10 controls to maintain the high-speed-rotation-side connection state even after the predetermined time elapses. In this way, by maintaining the high-speed-rotation-side connection state for a predetermined time after switching to the high-speed-rotation-side connection state, a switching operation can be eliminated when transitioning to the stance phase. On the other hand, when the phase determination unit 11 does not acquire the stance phase during the predetermined time, the control unit 10 switches the state to the disconnection state after the predetermined time elapses. Accordingly, when there is no transition from the swing phase to the stance phase, a failure caused by maintaining the high-speed-rotation-side connection state can be avoided.

In an example of FIG. 22, after the switching from the disconnection state to the high-speed-rotation-side connection state, the phase determination unit 11 determines the transition from the swing phase to the stance phase when a sensor value of the load sensor 272 is -110 [N] or less during 300 [ms], and control is performed to maintain the high-speed-rotation-side connection state even after the elapse of 300 [ms].

### (Trip Prevention Control)

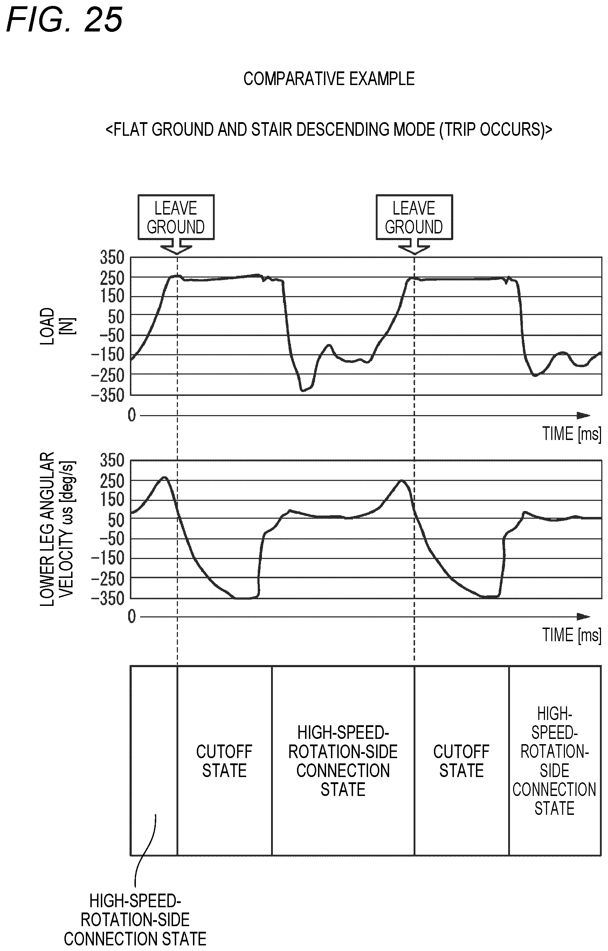

Meanwhile, in the flat ground and stair descending mode, as shown in a comparative example of FIG. 25, since the transition from the stance phase to the swing phase is determined when the electric prosthetic leg 1 leaves the ground, and the first connection and disconnection mechanism 210 and the second connection and disconnection mechanism 220 are switched to the disconnection state after the determination, a toe may be caught on the ground, that is, a so-called trip may occur, causing the user of the electric prosthetic leg 1 to feel uncomfortable. The trip may occur when free rotation of the knee joint mechanism 130 is hindered when leaving the ground, since the high-speed-rotation-side connection state is maintained to prevent giving way during the stance phase. The trip often occurs when the toe is caught on the ground, but may also occur when the heel is caught on the ground.

The electric prosthetic leg 1 is provided such that the second formed angle θ2 (knee-back angle) becomes substantially the maximum formed angle (185 [deg]), in other words, the knee angle θ is substantially the minimum formed angle (-5 [deg]) when transitioning from the stance phase to the swing phase, that is, at the time of leaving the ground during walking on the flat ground, but the trip may occur at this time.

Therefore, the phase transition prediction unit 12 predicts the transition of the electric prosthetic leg 1 from the stance phase to the swing phase to acquire the transition, and the motor control unit 13 controls to switch the first connection and disconnection mechanism 210 and the second connection and disconnection mechanism 220 from the high-speed-rotation-side connection state to the disconnection state before the electric prosthetic leg 1 starts to transition from the stance phase to the swing phase.

Specifically, in a case where the electric prosthetic leg 1 is in the stance phase and the motor control unit 13 controls the first connection and disconnection mechanism 210 and the second connection and disconnection mechanism 220 to the high-speed-rotation-side connection state, the motor control unit 13 controls to switch the first connection and disconnection mechanism 210 and the second connection and disconnection mechanism 220 from the high-speed-rotation-side connection state to the disconnection state when the following condition (Q1) is satisfied, preferably at least one of (Q2) and (Q3) is further satisfied.

### (Q1)

The phase transition prediction unit 12 detects that the second formed angle θ2 becomes equal to or larger than a threshold smaller than the maximum formed angle by a predetermined angle, in other words, the knee angle θ becomes equal to or smaller than a threshold.

### (Q2)

The thigh angle θt is within a third predetermined range.

### (Q3)

The lower leg angular velocity ωs is in a fourth predetermined range.

(Q1) is a condition for determining a time immediately before the occurrence of the trip, and (Q2) and (Q3) are conditions for recognizing the flat ground walking action (the stance phase of the electric prosthetic leg 1). Although the conditions (Q2) and/or (Q3) are not necessarily required, the trip can be more appropriately prevented by using these conditions as determination conditions.

The threshold of the second formed angle θ2 is, for example, 172 [deg]. In other words, the knee angle θ is 8 [deg]. When the second formed angle θ2 is equal to or larger than 172 [deg], in other words, when the knee angle θ is 8 [deg] or less, the occurrence of the trip before contacting the ground can be predicted, as shown in FIG. 22.

The third predetermined range is, for example, a positive range. That is, as described above, since the thigh angle θt takes a positive value when the above-knee member 120 is behind the vertical line VL2 (see (B) of FIG. 19), this condition makes it possible to determine that the electric prosthetic leg 1 is swung rearward in the flat ground walking action, that is, a later half of the stance phase in the flat ground walking action (see FIG. 22).

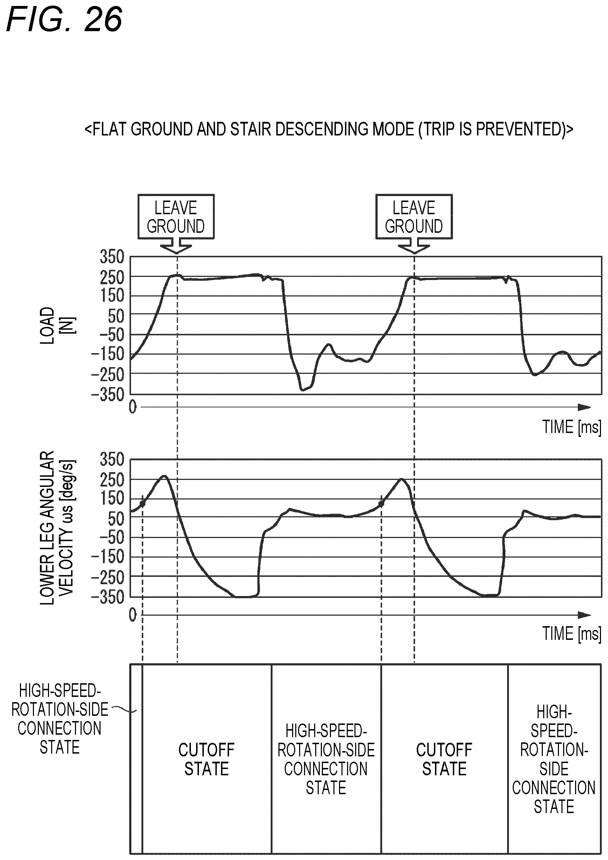

The fourth predetermined range is, for example, a range 100 [deg/s] or more. That is, as described above, since the lower leg angle θs takes a positive value when the below-knee member 110 is behind the vertical line VL1 (see (C) of FIG. 19), the lower leg angular velocity ωs being in the range of 100 [deg/s] or more means that a speed of swinging the below-knee member 110 rearward is high. Since the speed of swinging the below-knee member 110 rearward is high, it is possible to determine the later half of the stance phase in the flat ground walking action (see FIGs. 22 and 26).

From the graph of FIG. 22 showing a relationship of the knee angle θ, the thigh angle θt, the lower leg angle θs, and the load in the flat ground and stair descending mode, it can be seen that the thigh angle θt is in a positive range and the lower leg angular velocity ωs is 100 [deg/s] or more, immediately before the occurrence of the trip.

Therefore, when the thigh angle θt is in a positive range and the lower leg angular velocity ωs is 100 [deg/s] or more at a time before a time when the knee angle θ becomes substantially the minimum formed angle (the second formed angle θ2 (knee-back angle) is substantially the maximum formed angle of 185 degrees), and the phase transition prediction unit 12 detects that the knee angle θ is 8 degrees or less (the second formed angle θ2 is equal to or larger than 172 degrees), the control unit 10 predicts the transition of the electric prosthetic leg 1 from the stance phase to the swing phase, and controls to switch from the high-speed-rotation-side connection state to the disconnection state.

When the first connection and disconnection mechanism 210 and the second connection and disconnection mechanism 220 are in the disconnection state, the free rotation of the knee joint mechanism 130 is allowed, and the occurrence of the trip is prevented.

The control method in the stair ascending mode and the flat ground and stair descending mode can be realized by executing a program prepared in advance by a computer (processor). The program is stored in a storage medium readable by a computer, and is executed by being read from the storage medium. In addition, the program may be provided in a form stored in a non-transitory storage medium such as a flash memory, or may be provided via a network such as the Internet.

Although the various embodiments have been described above with reference to the drawings, it is needless to say that the present invention is not limited to these examples. It is apparent that those skilled in the art can conceive of various modifications and changes within the scope described in the claims, and it is understood that such modifications and changes naturally fall within the technical scope of the present invention. In addition, constituent elements in the embodiment described above may be freely combined without departing from the gist of the present invention.

For example, in the above embodiments, although prosthetic leg devices (electric prosthetic leg) applied to a knee joint as embodiments of the joint device of the present invention in which the connection and disconnection device is used are described, the present invention is not limited thereto, and may be a prosthetic limb device (electric prosthetic limb) applied to an elbow joint, and the wearer may be an animal other than a human, or a robot. When applied to an elbow joint, the below-knee member 110 in the above embodiment becomes a distal end side of a wearer with respect to the above-knee member 120, that is, a forearm.

In the present description, at least the following matters are described. In the parentheses, the corresponding constituent elements and the like in the above embodiment are shown, but the present invention is not limited thereto.
(1) A joint device (electric prosthetic leg 1) including:
   a first member (below-knee member 110);
   a second member (above-knee member 120);
   a coupling portion (knee joint mechanism 130) configured to couple the first member and the second member such that a formed angle (second formed angle θ2) formed between the first member and the second member is variable; and
   an enlarging and reducing device (enlarging and reducing device 200) capable of enlarging and reducing the formed angle formed between the first member and the second member, in which
   the enlarging and reducing device includes a power source (motor M), and a power transmission unit (transmission T) configured to transmit power of the power source,
   the power transmission unit has:
      a first power transmission path (first transmission mechanism T1) for transmitting the power at a first transmission ratio; and
      a second power transmission path (second transmission mechanism T2) for transmitting the power at a second transmission ratio different from the first transmission ratio,
   the enlarging and reducing device further includes:
      a first connection and disconnection mechanism (first connection and disconnection mechanism 210) configured to switch between connection and disconnection of power in the first power transmission path;
      a second connection and disconnection mechanism (second connection and disconnection mechanism 220) configured to switch between connection and disconnection of power in the second power transmission path; and
      a control unit (control unit 10) configured to control the power source, the first connection and disconnection mechanism, and the second connection and disconnection mechanism,
   the joint device is provided to transition between a weighted state (stance phase) where a weight applied from an outside is received and a non-weighted state (swing phase) where no weight is received, and
   the control unit,
      (A) when the joint device is in the weighted state,
         controls to be in a first connection state (high-torque-side connection state) where any one of the first connection and disconnection mechanism and the second connection and disconnection mechanism is connected, and
      (B) when the joint device is in the non-weighted state,
         controls to be in a disconnection state (disconnection state) where the first connection and disconnection mechanism and the second connection and disconnection mechanism are disconnected, or
         controls to be in a second connection state (high-speed-rotation-side connection state) where the other of the first connection and disconnection mechanism and the second connection and disconnection mechanism is connected.

According to (1), the joint device appropriately switches the states of the first connection and disconnection mechanism and the second connection and disconnection mechanism between the weighted state where a weight applied from the outside is received and the non-weighted state where no weight is received, and thus convenience of the joint device is improved.

(2) The joint device according to (1), including:
a weight acquisition unit (phase transition prediction unit 12) configured to acquire a weight which the joint device receives, or acquire the weighted state or the non-weighted state, in which
the control unit,
   (a) when the weight acquisition unit acquires a transition from the non-weighted state to the weighted state,
      controls to switch from the disconnection state or the second connection state to the first connection state, and
   (b) when the weight acquisition unit acquires a transition from the weighted state to the non-weighted state,
      controls to switch from the first connection state to the disconnection state or the second connection state.

According to (2), by appropriately switching the states of the first connection and disconnection mechanism and the second connection and disconnection mechanism when the weight acquisition unit acquires the state transition, a switching operation can be performed smoothly.

(3) The joint device according to (2), in which
the control unit,
   (a) when the weight acquisition unit acquires the transition from the non-weighted state to the weighted state,
      controls to switch from the disconnection state to the first connection state,
the weight acquisition unit predicts the transition from the non-weighted state to the weighted state to acquire the transition, and
the control unit controls to switch from the disconnection state to the first connection state before the transition from the non-weighted state to the weighted state is started.

According to (3), the weight acquisition unit predicts the transition from the non-weighted state to the weighted state to acquire the transition, the switching operation can be performed more smoothly.

(4) The joint device according to any one of (1) to (3), in which
the formed angle is defined such that an angle on one side in one circumference centered on a coupling axis of the coupling portion is a first formed angle (first formed angle θ1), and an angle on an other side is a second formed angle (second formed angle θ2),
when a smaller minimum formed angle in a range in which the first member and the second member move relative to each other is, out of the first formed angle and the second formed angle, defined as the second formed angle,
the second formed angle is provided to take a value in a range from the minimum formed angle (60 degrees) to a maximum formed angle (185 degrees), and
the joint device is provided such that the second formed angle becomes substantially the maximum formed angle when the joint device transitions from the non-weighted state to the weighted state.

According to (4), a terminal impact may occur when the non-weighted state transitions to the weighted state.

(5) The joint device according to (4), including:
a weight acquisition unit (phase transition prediction unit 12) configured to acquire a weight which the joint device receives, or acquire the weighted state or the non-weighted state, in which
in a case where the joint device is in the non-weighted state and the control unit controls to be in the disconnection state,
when the weight acquisition unit acquires that the second formed angle becomes equal to or larger than a threshold smaller than the maximum formed angle by a predetermined angle (the second formed angle θ2 is equal to or larger than 172 degrees),
the control unit controls to switch from the disconnection state to the first connection state.

According to (5), the terminal impact can be prevented.

(6) The joint device according to (5), in which
in a case where the joint device is in the non-weighted state and the control unit controls to be in the disconnection state,
when the weight acquisition unit acquires that the second formed angle becomes equal to or larger than the threshold, and an angle (thigh angle θt) formed between the second member and a reference line (vertical line VL2) passing through a pivoting axis (hip joint 124) of a first portion (thigh 123) of a wearer and a second portion (upper body) pivoting relative to the first portion is within a first predetermined range (negative range),
the control unit controls to switch from the disconnection state to the first connection state.

According to (6), the terminal impact can be more appropriately prevented.

(7) The joint device according to (5) or (6), in which
in a case where the joint device is in the non-weighted state and the control unit controls to be in the disconnection state,
when the weight acquisition unit acquires that the second formed angle becomes equal to or larger than the threshold, and an angular velocity (lower leg angular velocity ωs) of another angle (lower leg angle θs) formed between the first member and another reference line (vertical line VL1) passing through another pivoting axis (coupling axis 135) of the first member and the second member is within a second predetermined range (a range of -100 [deg/s]),
the control unit controls to switch from the disconnection state to the first connection state.

According to (7), the terminal impact can be more appropriately prevented.

(8) The joint device according to any one of (5) to (7), in which
the control unit controls to maintain the first connection state for a predetermined time (300 [ms]) after the switching from the disconnection state to the first connection state.

According to (8), when there is no transition from the non-weighted state to the weighted state, a failure caused by maintaining the first connection state can be avoided.

(9) The joint device according to (8), including:
a weight transition acquisition unit (phase determination unit 11) configured to acquire that the joint device becomes the weighted state, in which
when the weight transition acquisition unit acquires, during the predetermined time, that the joint device becomes the weighted state,
the control unit controls to maintain the first connection state even after the predetermined time elapses.

According to (9), by maintaining the first connection state for a predetermined time after switching to the first connection state, the switching operation can be eliminated when transitioning to the weighted state.

(10) The joint device according to (2) or (3), in which
the control unit,
   (b) when the weight acquisition unit acquires the transition from the weighted state to the non-weighted state,
   controls to switch from the first connection state to the disconnection state,
the weight acquisition unit predicts the transition from the weighted state to the non-weighted state to acquire the transition, and
the control unit controls to switch from the first connection state to the disconnection state before the transition from the weighted state to the non-weighted state is started.

According to (10), the weight acquisition unit predicts the transition from the weighted state to the non-weighted state to acquire the transition, and thus the switching operation can be performed more smoothly.

(11) The joint device according to any one of (1) to (10), in which
the formed angle is defined such that an angle on one side in one circumference centered on a coupling axis of the coupling portion is a first formed angle (first formed angle θ1) and an angle on an other side is a second formed angle (second formed angle θ2),
when a smaller minimum formed angle in a range in which the first member and the second member move relative to each other is, out of the first formed angle and the second formed angle, defined as the second formed angle,
the second formed angle is provided to take a value in a range from the minimum formed angle (60 degrees) to a maximum formed angle (185 degrees), and
the joint device is provided such that the second formed angle becomes substantially the maximum formed angle when the joint device transitions from the weighted state to the non-weighted state.

According to (11), for example, in a case where the joint device is used as a prosthetic leg device, trip may occur when a transitioning from the weighted state to the non-weighted state occurs.

(12) The joint device according to (11), including:
a weight acquisition unit (phase transition prediction unit 12) configured to acquire a weight which the joint device receives, or acquire the weighted state or the non-weighted state, in which
in a case where the joint device is in the weighted state and the control unit controls to be in the first connection state,
when the weight acquisition unit acquires that the second formed angle becomes equal to or larger than a threshold smaller than the maximum formed angle by a predetermined angle (the second formed angle θ2 becomes equal to or larger than 172 degrees),
the control unit controls to switch from the first connection state to the disconnection state.

According to (12), the trip can be prevented.

(13) The joint device according to (12), in which
in a case where the joint device is in the weighted state and the control unit controls to be in the first connection state,
when the weight acquisition unit acquires that the second formed angle becomes equal to or larger than the threshold, and an angle (thigh angle θt) formed between the second member and a reference line (vertical line VL2) passing through a pivoting axis (hip joint 124) of a first portion (thigh 123) of a wearer and a second portion (upper body) pivoting relative to the first portion is within a third predetermined range (positive range),
the control unit controls to switch from the first connection state to the disconnection state.

According to (13), the trip can be prevented more appropriately.

(14) The joint device according to (12) or (13), in which
in a case where the joint device is in the weighted state and the control unit controls to be in the first connection state,
when the weight acquisition unit acquires that the second formed angle becomes equal to or larger than the threshold, and an angular velocity (lower leg angular velocity ωs) of another angle (lower leg angle θs) formed between the first member and another reference line (vertical line VL1) passing through another pivoting axis (coupling axis 135) of the first member and the second member is within a fourth predetermined range (a range of 100 [deg/s] or more),
the control unit controls to switch from the first connection state to the disconnection state.

According to (14), the trip can be prevented more appropriately.

(15) The joint device according to any one of (1) to (14), in which
the enlarging and reducing device is an expansion-contraction device (expansion-contraction device 140), and
the expansion-contraction device includes an expansion-contraction unit (spindle unit SP) capable of enlarging and reducing the formed angle by expansion and contraction, in which one end side thereof in an extending direction is mechanically connected to the first member and an other end side thereof is mechanically connected to the second member.

According to (15), the formed angle can be enlarged and reduced by the expansion and contraction of the expansion-contraction unit.

(16) The joint device according to (15), in which
the expansion-contraction unit is disposed on a side opposite to the power source with respect to the power transmission unit on a transmission path of the power.

According to (16), the expansion-contraction unit can be operated by the power transmitted through the power transmission unit.

(17) The joint device according to (15) or (16), in which
the power source is configured to output rotational power, and
the expansion-contraction unit includes a motion conversion mechanism (spindle unit SP) configured to convert rotational power output from the power source into translational motion.

According to (17), the expansion-contraction unit can be expanded and contracted by the motion conversion mechanism.

(18) The joint device according to (17), in which
the motion conversion mechanism includes a shaft member (spindle 173), and a cylindrical member (sleeve 174) configured to perform translational motion along an axis of the shaft member according to rotation of the shaft member.

According to (18), the motion conversion mechanism can be implemented with a simple configuration.

(19) The joint device according to any one of (1) to (14), in which
when the first transmission ratio is defined as a ratio of a post-transmission rotation speed with respect to a pre-transmission rotation speed which is a rotation speed on a power source side in the first power transmission path relative to a first transmission unit, and
the second transmission ratio is defined as a ratio of a post-transmission rotation speed with respect to a pre-transmission rotation speed which is a rotation speed on the power source side in the second power transmission path relative to a second transmission unit,
the first transmission ratio is configured to be smaller than the second transmission ratio, and
the control unit,
   (A) when the joint device is in the weighted state,
      controls to be in the first connection state where the first connection and disconnection mechanism is connected, and
   (B) when the joint device is in the non-weighted state,
      controls to be in the second connection state where the second connection and disconnection mechanism is connected.

According to (19), for example, when the joint device is used as a prosthetic leg device, stair ascending is smoothly performed by switching between the first connection state and the second connection state in the weighted state and the non-weighted state.

(20) The joint device according to (19), in which
the control unit further controls the power source, and
the control unit,
   (A) when the first connection and disconnection mechanism and the second connection and disconnection mechanism are in the first connection state,
      controls the power source based on a torque target value which is a target value of a torque related to a torque for the enlarging and reducing device to enlarge or reduce the formed angle, and
   (B) when the first connection and disconnection mechanism and the second connection and disconnection mechanism are in the second connection state,
      controls the power source based on a position target value which is a target value of a position related to the formed angle enlarged or reduced by the enlarging and reducing device.

According to (20), the stair ascending is more smoothly performed.

(21) The joint device according to any one of (1) to (20), in which
the joint device is attached to a wearer such that the first member is situated at a distal end side of the wearer than the second member, and
the joint device is a prosthetic limb device provided such that the coupling portion functions as a joint of the wearer.

According to (21), a smooth bending operation and a smooth stretching operation can be performed.

(22) The joint device according to (21), in which
the prosthetic limb device is a prosthetic leg device to be attached to a leg of the wearer.

According to (22), the smooth bending operation and the smooth stretching operation of a prosthetic leg can be performed.

(23) The joint device according to (22), in which
the prosthetic leg device is provided such that the second member is attached to a thigh of the leg and the coupling portion is provided to function as a knee joint between the thigh and a lower leg.

According to (23), smooth walking can be performed.

(24) The joint device according to (22) or (23), in which
the control unit,
(B) in a case of walking to advance on a flat ground, or in a case of walking to descend stairs,
when the joint device is in the non-weighted state
   controls to be in the disconnection state.

According to (24), walking on the flat ground and stair descending can be smoothly performed.

(25) The joint device according to any one of (22) to (24), in which
the control unit,
   (B) in a case of walking to ascend stairs,
   when the joint device is in the non-weighted state,
      controls to be in the second connection state.

According to (25), walking on the flat ground and stair ascending can be smoothly performed.

(26) A knee joint device (electric prosthetic leg 1) including:
a first member (below-knee member 110);
a second member (above-knee member 120);
a coupling portion (knee joint mechanism 130) configured to couple the first member and the second member such that a formed angle formed between the first member and the second member is variable; and
an enlarging and reducing device (enlarging and reducing device 200) capable of enlarging and reducing the formed angle formed between the first member and the second member, in which
the enlarging and reducing device includes a power source (motor M), and a power transmission unit (transmission T) configured to transmit power of the power source,
the power transmission unit has:
   a first power transmission path (first transmission mechanism T1) for transmitting the power at a first transmission ratio, and
   a second power transmission path (second transmission mechanism T2) for transmitting the power at a second transmission ratio different from the first transmission ratio,
the enlarging and reducing device further includes:
   a first connection and disconnection mechanism (first connection and disconnection mechanism 210) configured to switch between connection and disconnection of power in the first power transmission path,
   a second connection and disconnection mechanism (second connection and disconnection mechanism 220) configured to switch between connection and disconnection of power in the second power transmission path, and
   a control unit (control unit 10) configured to control the power source, the first connection and disconnection mechanism, and the second connection and disconnection mechanism,
the knee joint device is provided to transition between a stance state where a load from a wearer of the knee joint device is received and a swing state where no load is received, and
the control unit,
   (A) when the joint device is in the stance state,
      controls to be in a first connection state where any one of the first connection and disconnection mechanism and the second connection and disconnection mechanism is connected, and
   (B) when the joint device is in the swing state,
      controls to be in a disconnection state where the first connection and disconnection mechanism and the second connection and disconnection mechanism are disconnected, or
      controls to be in a second connection state where the other of the first connection and disconnection mechanism and the second connection and disconnection mechanism is connected.

According to (26), the knee joint device appropriately switches the states of the first connection and disconnection mechanism and the second connection and disconnection mechanism between the weighted state where a weight applied from the outside is received and the non-weighted state where no weight is received, and thus convenience of the knee joint device is improved.

(27) A joint device control method for controlling a joint device (electric prosthetic leg 1) including:
a first member (below-knee member 110),
a second member (above-knee member 120),
a coupling portion (knee joint mechanism 130) configured to couple the first member and the second member such that a formed angle formed between the first member and the second member is variable, and
an enlarging and reducing device (enlarging and reducing device 200) capable of enlarging and reducing the formed angle formed between the first member and the second member,
the enlarging and reducing device includes a power source (motor M), and a power transmission unit (transmission T) configured to transmit power of the power source,
the power transmission unit has:
   a first power transmission path (first transmission mechanism T1) for transmitting the power at a first transmission ratio; and
   a second power transmission path (second transmission mechanism T2) for transmitting the power at a second transmission ratio different from the first transmission ratio,
the enlarging and reducing device further includes:
   a first connection and disconnection mechanism (first connection and disconnection mechanism 210) configured to switch between connection and disconnection of power in the first power transmission path; and
   a second connection and disconnection mechanism (second connection and disconnection mechanism 220) configured to switch between connection and disconnection of power in the second power transmission path,
the joint device is provided to transition between a weighted state where a weight applied from an outside is received and a non-weighted state where no weight is received, and
the control method includes the steps of:
   (A) when the joint device is in the weighted state,
      controlling to be in a first connection state where any one of the first connection and disconnection mechanism and the second connection and disconnection mechanism is connected; and
   (B) when the joint device is in the non-weighted state,
      controlling to be in a disconnection state where the first connection and disconnection mechanism and the second connection and disconnection mechanism are disconnected, or
      controlling to be in a second connection state where an other of the first connection and disconnection mechanism and the second connection and disconnection mechanism is connected.

According to (27), the joint device appropriately switches the states of the first connection and disconnection mechanism and the second connection and disconnection mechanism between the weighted state where a weight applied from the outside is received and the non-weighted state where no weight is received, and thus the convenience of the joint device is improved.

(28) A joint device control program for controlling a joint device (electric prosthetic leg 1) including:
a first member (below-knee member 110);
a second member (above-knee member 120);
a coupling portion (knee joint mechanism 130) configured to couple the first member and the second member such that a formed angle formed between the first member and the second member is variable; and
an enlarging and reducing device (enlarging and reducing device 200) capable of enlarging and reducing the formed angle formed between the first member and the second member, in which
the enlarging and reducing device includes a power source (motor M), and a power transmission unit (transmission T) configured to transmit power of the power source,
the power transmission unit has:
   a first power transmission path (first transmission mechanism T1) for transmitting the power at a first transmission ratio; and
   a second power transmission path (second transmission mechanism T2) for transmitting the power at a second transmission ratio different from the first transmission ratio,
the enlarging and reducing device further includes:
   a first connection and disconnection mechanism (first connection and disconnection mechanism 210) configured to switch between connection and disconnection of power in the first power transmission path; and
   a second connection and disconnection mechanism (second connection and disconnection mechanism 220) configured to switch between connection and disconnection of power in the second power transmission path, and
the joint device is provided to transition between a weighted state where a weight applied from an outside is received and a non-weighted state where no weight is received, and
the control program causes a computer to perform the steps of:
   (A) when the joint device is in the weighted state, controlling to be in a first connection state where any one of the first connection and disconnection mechanism and the second connection and disconnection mechanism is connected; and
   (B) when the joint device is in the non-weighted state,
      controlling to be in a disconnection state where the first connection and disconnection mechanism and the second connection and disconnection mechanism are disconnected, or
      controlling to be in a second connection state where an other of the first connection and disconnection mechanism and the second connection and disconnection mechanism is connected.

According to (28), the joint device appropriately switches the states of the first connection and disconnection mechanism and the second connection and disconnection mechanism between the weighted state where a weight applied from the outside is received and the non-weighted state where no weight is received, and thus the convenience of the joint device is improved.

(29) A computer-readable storage medium storing the control program according to (28).

According to (29), the joint device appropriately switches the states of the first connection and disconnection mechanism and the second connection and disconnection mechanism between the weighted state where a weight applied from the outside is received and the non-weighted state where no weight is received, and thus the convenience of the joint device is improved.

The present application is based on a Japanese Patent Application (Japanese Patent Application No. 2022-142465) filed on September 7, 2022, and the contents thereof are incorporated herein by reference.

### REFERENCE SIGNS LIST

1: electric prosthetic leg (joint device, knee joint device)
10: control unit
11: phase determination unit (weight transition acquisition unit)
12: phase transition prediction unit (weight acquisition unit)
110: below-knee member (first member)
120: above-knee member (second member)
130: knee joint mechanism (coupling portion)
135: coupling axis (another pivoting axis)
140: expansion-contraction device
173: spindle (shaft member)
174: sleeve (cylindrical member)
200: enlarging and reducing device
210: first connection and disconnection mechanism
220: second connection and disconnection mechanism
M: motor (power source)
SP: spindle unit (motion conversion mechanism, expansion-contraction unit)
T: transmission (power transmission unit)
T1: first transmission mechanism (first power transmission path)
T2: second transmission mechanism (second power transmission path)
VL1: vertical line (another reference line)
VL2: vertical line (reference line)
θ2: second formed angle (angle)
θs: lower leg angle (another angle)
Θt: thigh angle

## Claims

1. A joint device comprising:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the power transmission unit has:
a first power transmission path for transmitting the power at a first transmission ratio; and
a second power transmission path for transmitting the power at a second transmission ratio different from the first transmission ratio,
the enlarging and reducing device further includes:
a first connection and disconnection mechanism configured to switch between connection and disconnection of power in the first power transmission path;
a second connection and disconnection mechanism configured to switch between connection and disconnection of power in the second power transmission path; and
a control unit configured to control the power source, the first connection and disconnection mechanism, and the second connection and disconnection mechanism,
the joint device is provided to transition between a weighted state where a weight applied from an outside is received and a non-weighted state where no weight is received, and
the control unit,
(A) when the joint device is in the weighted state,
controls to be in a first connection state where any one of the first connection and disconnection mechanism and the second connection and disconnection mechanism is connected, and
(B) when the joint device is in the non-weighted state,
controls to be in a disconnection state where the first connection and disconnection mechanism and the second connection and disconnection mechanism are disconnected, or
controls to be in a second connection state where an other of the first connection and disconnection mechanism and the second connection and disconnection mechanism is connected.

2. The joint device according to claim 1, comprising:
a weight acquisition unit configured to acquire a weight which the joint device receives, or acquire the weighted state or the non-weighted state, wherein
the control unit,
(a) when the weight acquisition unit acquires a transition from the non-weighted state to the weighted state,
controls to switch from the disconnection state or the second connection state to the first connection state, and
(b) when the weight acquisition unit acquires a transition from the weighted state to the non-weighted state,
controls to switch from the first connection state to the disconnection state or the second connection state.

3. The joint device according to claim 2, wherein
the control unit,
(a) when the weight acquisition unit acquires the transition from the non-weighted state to the weighted state,
controls to switch from the disconnection state to the first connection state, and
the weight acquisition unit predicts the transition from the non-weighted state to the weighted state to acquire the transition, and
the control unit controls to switch from the disconnection state to the first connection state before the transition from the non-weighted state to the weighted state is started.

4. The joint device according to any one of claims 1 to 3, wherein
the formed angle is defined such that an angle on one side in one circumference centered on a coupling axis of the coupling portion is a first formed angle, and an angle on an other side is a second formed angle,
when a smaller minimum formed angle in a range in which the first member and the second member move relative to each other is, out of the first formed angle and the second formed angle, defined as the second formed angle,
the second formed angle is provided to take a value in a range from the minimum formed angle to a maximum formed angle, and
the joint device is provided such that the second formed angle becomes substantially the maximum formed angle when the joint device transitions from the non-weighted state to the weighted state.

5. The joint device according to claim 4, comprising:
a weight acquisition unit configured to acquire a weight which the joint device receives, or acquire the weighted state or the non-weighted state, wherein
in a case where the joint device is in the non-weighted state and the control unit controls to be in the disconnection state,
when the weight acquisition unit acquires that the second formed angle becomes equal to or larger than a threshold smaller than the maximum formed angle by a predetermined angle,
the control unit controls to switch from the disconnection state to the first connection state.

6. The joint device according to claim 5, wherein
in a case where the joint device is in the non-weighted state and the control unit controls to be in the disconnection state,
when the weight acquisition unit acquires that the second formed angle becomes equal to or larger than the threshold, and a formed angle formed between the second member and a reference line passing through a pivoting axis of a first portion of a wearer and a second portion pivoting relative to the first portion is within a first predetermined range,
the control unit controls to switch from the disconnection state to the first connection state.

7. The joint device according to claim 5 or 6, wherein
in a case where the joint device is in the non-weighted state and the control unit controls to be in the disconnection state,
when the weight acquisition unit acquires that the second formed angle becomes equal to or larger than the threshold, and an angular velocity of another formed angle formed between the first member and another reference line passing through another pivoting axis of the first member and the second member is within a second predetermined range,
the control unit controls to switch from the disconnection state to the first connection state.

8. The joint device according to any one of claims 5 to 7, wherein
the control unit controls to maintain the first connection state for a predetermined time after the switching from the disconnection state to the first connection state.

9. The joint device according to claim 8, comprising:
a weight transition acquisition unit configured to acquire that the joint device becomes the weighted state, wherein
when the weight transition acquisition unit acquires, during the predetermined time, that the joint device becomes the weighted state,
the control unit controls to maintain the first connection state even after the predetermined time elapses.

10. The joint device according to claim 2 or 3, wherein
the control unit,
(b) when the weight acquisition unit acquires the transition from the weighted state to the non-weighted state,
controls to switch from the first connection state to the disconnection state,
the weight acquisition unit predicts the transition from the weighted state to the non-weighted state to acquire the transition, and
the control unit controls to switch from the first connection state to the disconnection state before the transition from the weighted state to the non-weighted state is started.

11. The joint device according to any one of claims 1 to 10, wherein
the formed angle is defined such that an angle on one side in one circumference centered on a coupling axis of the coupling portion is a first formed angle, and an angle on an other side is a second formed angle,
when a smaller minimum formed angle in a range in which the first member and the second member move relative to each other is, out of the first formed angle and the second formed angle, defined as the second formed angle,
the second formed angle is provided to take a value in a range from the minimum formed angle to a maximum formed angle, and
the joint device is provided such that the second formed angle becomes substantially the maximum formed angle when the joint device transitions from the weighted state to the non-weighted state.

12. The joint device according to claim 11, comprising:
a weight acquisition unit configured to acquire a weight which the joint device receives, or acquire the weighted state or the non-weighted state, wherein
in a case where the joint device is in the weighted state and the control unit controls to be in the first connection state,
when the weight acquisition unit acquires that the second formed angle becomes equal to or larger than a threshold smaller than the maximum formed angle by a predetermined angle,
the control unit controls to switch from the first connection state to the disconnection state.

13. The joint device according to claim 12, wherein
in a case where the joint device is in the weighted state and the control unit controls to be in the first connection state,
when the weight acquisition unit acquires that the second formed angle becomes equal to or larger than the threshold, and a formed angle formed between the second member and a reference line passing through a pivoting axis of a first portion of a wearer and a second portion pivoting relative to the first portion is within a third predetermined range,
the control unit controls to switch from the first connection state to the disconnection state.

14. The joint device according to claim 12 or 13, wherein
in a case where the joint device is in the weighted state and the control unit controls to be in the first connection state,
when the weight acquisition unit acquires that the second formed angle becomes equal to or larger than the threshold, and an angular velocity of another formed angle formed between the first member and another reference line passing through another pivoting axis of the first member and the second member is within a fourth predetermined range,
the control unit controls to switch from the first connection state to the disconnection state.

15. The joint device according to any one of claims 1 to 14, wherein
the enlarging and reducing device is an expansion-contraction device, and
the expansion-contraction device includes an expansion-contraction unit capable of enlarging and reducing the formed angle by expansion and contraction, in which one end side thereof in an extending direction is mechanically connected to the first member and an other end side thereof is mechanically connected to the second member.

16. The joint device according to claim 15, wherein
the expansion-contraction unit is disposed on a side opposite to the power source with respect to the power transmission unit on a transmission path of the power.

17. The joint device according to claim 15 or 16, wherein
the power source is configured to output rotational power, and
the expansion-contraction unit includes a motion conversion mechanism configured to convert rotational power output from the power source into translational motion.

18. The joint device according to claim 17, wherein
the motion conversion mechanism includes a shaft member, and a cylindrical member configured to perform translational motion along an axis of the shaft member according to rotation of the shaft member.

19. The joint device according to any one of claims 1 to 14, wherein
when the first transmission ratio is defined as a ratio of a post-transmission rotation speed with respect to a pre-transmission rotation speed which is a rotation speed on a power source side in the first power transmission path relative to a first transmission unit, and
the second transmission ratio is defined as a ratio of a post-transmission rotation speed with respect to a pre-transmission rotation speed which is a rotation speed on the power source side in the second power transmission path relative to a second transmission unit,
the first transmission ratio is configured to be smaller than the second transmission ratio, and
the control unit,
(A) when the joint device is in the weighted state,
controls to be in the first connection state where the first connection and disconnection mechanism is connected, and
(B) when the joint device is in the non-weighted state,
controls to be in the second connection state where the second connection and disconnection mechanism is connected.

20. The joint device according to claim 19, wherein
the control unit further controls the power source, and
the control unit,
(A) when the first connection and disconnection mechanism and the second connection and disconnection mechanism are in the first connection state,
controls the power source based on a torque target value which is a target value of a torque related to a torque for the enlarging and reducing device to enlarge or reduce the formed angle, and
(B) when the first connection and disconnection mechanism and the second connection and disconnection mechanism are in the second connection state,
controls the power source based on a position target value which is a target value of a position related to the formed angle enlarged or reduced by the enlarging and reducing device.

21. The joint device according to any one of claims 1 to 20, wherein
the joint device is attached to a wearer such that the first member is situated at a distal end side of the wearer than the second member, and
the joint device is a prosthetic limb device provided such that the coupling portion functions as a joint of the wearer.

22. The joint device according to claim 21, wherein
the prosthetic limb device is a prosthetic leg device to be attached to a leg of the wearer.

23. The joint device according to claim 22, wherein
the prosthetic leg device is provided such that the second member is attached to a thigh of the leg, and the coupling portion is provided to function as a knee joint between the thigh and a lower leg.

24. The joint device according to claim 22 or 23, wherein
the control unit,
(B) in a case of walking to advance on a flat ground, or in a case of walking to descend stairs,
when the joint device is in the non-weighted state
controls to be in the disconnection state.

25. The joint device according to any one of claims 22 to 24, wherein
the control unit,
(B) in a case of walking to ascend stairs,
when the joint device is in the non-weighted state,
controls to be in the second connection state.

26. A knee joint device comprising:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the power transmission unit has:
a first power transmission path for transmitting the power at a first transmission ratio; and
a second power transmission path for transmitting the power at a second transmission ratio different from the first transmission ratio,
the enlarging and reducing device further includes:
a first connection and disconnection mechanism configured to switch between connection and disconnection of power in the first power transmission path;
a second connection and disconnection mechanism configured to switch between connection and disconnection of power in the second power transmission path; and
a control unit configured to control the power source, the first connection and disconnection mechanism, and the second connection and disconnection mechanism,
the knee joint device is provided to transition between a stance state where a load from a wearer of the knee joint device is received and a swing state where no load is received, and
the control unit,
(A) when the knee joint device is in the stance state,
controls to be in a first connection state where any one of the first connection and disconnection mechanism and the second connection and disconnection mechanism is connected, and
(B) when the knee joint device is in the swing state,
controls to be in a disconnection state where the first connection and disconnection mechanism and the second connection and disconnection mechanism are disconnected, or
controls to be in a second connection state where an other of the first connection and disconnection mechanism and the second connection and disconnection mechanism is connected.

27. A joint device control method for controlling a joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the power transmission unit has:
a first power transmission path for transmitting the power at a first transmission ratio; and
a second power transmission path for transmitting the power at a second transmission ratio different from the first transmission ratio,
the enlarging and reducing device further includes:
a first connection and disconnection mechanism configured to switch between connection and disconnection of power in the first power transmission path; and
a second connection and disconnection mechanism configured to switch between connection and disconnection of power in the second power transmission path,
the joint device is provided to transition between a weighted state where a weight applied from an outside is received and a non-weighted state where no weight is received, and
the control method comprises the steps of:
(A) when the joint device is in the weighted state,
controlling to be in a first connection state where any one of the first connection and disconnection mechanism and the second connection and disconnection mechanism is connected; and
(B) when the joint device is in the non-weighted state,
controlling to be in a disconnection state where the first connection and disconnection mechanism and the second connection and disconnection mechanism are disconnected, or
controlling to be in a second connection state where an other of the first connection and disconnection mechanism and the second connection and disconnection mechanism is connected.

28. A joint device control program for controlling a joint device including:
a first member;
a second member;
a coupling portion configured to couple the first member and the second member such that a formed angle formed between the first member and the second member is variable; and
an enlarging and reducing device capable of enlarging and reducing the formed angle formed between the first member and the second member, wherein
the enlarging and reducing device includes a power source, and a power transmission unit configured to transmit power of the power source,
the power transmission unit has:
a first power transmission path for transmitting the power at a first transmission ratio; and
a second power transmission path for transmitting the power at a second transmission ratio different from the first transmission ratio,
the enlarging and reducing device further includes:
a first connection and disconnection mechanism configured to switch between connection and disconnection of power in the first power transmission path; and
a second connection and disconnection mechanism configured to switch between connection and disconnection of power in the second power transmission path, and
the joint device is provided to transition between a weighted state where a weight applied from an outside is received and a non-weighted state where no weight is received, and
the control program causes a computer to perform the steps of:
(A) when the joint device is in the weighted state,
controlling to be in a first connection state where any one of the first connection and disconnection mechanism and the second connection and disconnection mechanism is connected; and
(B) when the joint device is in the non-weighted state,
controlling to be in a disconnection state where the first connection and disconnection mechanism and the second connection and disconnection mechanism are disconnected, or
controlling to be in a second connection state where an other of the first connection and disconnection mechanism and the second connection and disconnection mechanism is connected.

29. A computer-readable storage medium storing the control program according to claim 28.
